(19) Europäisches Patentamt
European Patent Office
Office européen des brevets

(11) **EP 3 068 754 B1**

(12) **EUROPÄISCHE PATENTSCHRIFT**

(45) Veröffentlichungstag und Bekanntmachung des Hinweises auf die Patenterteilung:
**31.01.2018 Patentblatt 2018/05**

(21) Anmeldenummer: **14795613.0**

(22) Anmeldetag: **05.11.2014**

(51) Int Cl.:
*C07C 45/35* [(2006.01)]   *C07C 47/22* [(2006.01)]
*C07C 51/25* [(2006.01)]   *C07C 57/04* [(2006.01)]

(86) Internationale Anmeldenummer:
**PCT/EP2014/073810**

(87) Internationale Veröffentlichungsnummer:
**WO 2015/067656 (14.05.2015 Gazette 2015/19)**

(54) **VERFAHREN ZUR HERSTELLUNG EINES UNGESÄTTIGTEN ALDEHYDS UND/ODER EINER UNGESÄTTIGTEN CARBONSÄURE**

PROCESS FOR PREPARING AN UNSATURATED ALDEHYDE AND/OR AN UNSATURATED CARBOXYLIC ACID

PROCÉDÉ DE PRODUCTION D'UN ALDÉHYDE INSATURÉ ET/OU D'UN ACIDE CARBOXYLIQUE INSATURÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.11.2013 EP 13192279**

(43) Veröffentlichungstag der Anmeldung:
**21.09.2016 Patentblatt 2016/38**

(73) Patentinhaber: **BASF SE**
**67056 Ludwigshafen am Rhein (DE)**

(72) Erfinder:
• **MACHT, Josef**
**67056 Ludwigshafen (DE)**
• **WALSDORFF, Christian**
**67061 Ludwigshafen (DE)**
• **DOBNER, Cornelia Katharina**
**67071 Ludwigshafen (DE)**
• **WELKER-NIEUWOUDT, Cathrin Alexandra**
**67134 Birkenheide (DE)**
• **HAMMON, Ulrich**
**68163 Mannheim (DE)**

(74) Vertreter: **BASF IP Association**
**BASF SE**
**G-FLP-C006**
**67056 Ludwigshafen (DE)**

(56) Entgegenhaltungen:
**EP-A2- 1 350 566     WO-A2-02/24620**

• **DATABASE WPI Week 201401 Thomson Scientific, London, GB; AN 2013-K77167 XP002734674, & CN 102 992 979 A (CHINA NATIONAL PETROLEUM) 27. März 2013 (2013-03-27)**

**Beschreibung**

Derzeit erfolgt die großtechnische Herstellung von $\alpha,\beta$-ungesättigten Aldehyden und/oder $\alpha,\beta$-ungesättigten Carbonsäuren, insbesondere von Acrolein und Acrylsäure, im Wesentlichen durch heterogen katalysierte Partialoxidation von Alkenen (siehe beispielsweise die DE-A 103 36 386 zur Herstellung von Acrylsäure durch Partialoxidation von Propen).

Die Reaktion ist stark exotherm, wodurch sich im Katalysatorbett entlang des Gasstroms ein Temperaturprofil einstellt, das durch eine höchste lokale Temperatur gekennzeichnet ist.

Ist diese Temperatur hoch, so geht dies mit einer verstärkten Oxidation einher. Dadurch verringert sich die Ausbeute der Wertprodukte (an $\alpha,\beta$-ungesättigtem Aldehyd und $\alpha,\beta$-ungesättigter Carbonsäure).

Bei hohen Temperaturen wird der Katalysator außerdem schneller deaktiviert. Die Deaktivierung ist in Bereichen mit besonders hoher Temperatur besonders stark ausgeprägt. Auch die fortschreitende Deaktivierung des Katalysators verringert die Ausbeute der Wertprodukte.

Im Stand der Technik wurden Verfahren zur Partialoxidation von Alkenen zu $\alpha,\beta$-ungesättigten Aldehyden und $\alpha,\beta$-ungesättigten Carbonsäuren mit erhöhter Wertproduktausbeute vorgeschlagen. Unter anderem wurden strukturierte Katalysatorschüttungen entlang der Strömungsrichtung der Gase vorgeschlagen.

Die EP 1 350 566 B1 offenbart ein Verfahren zur Herstellung eines ungesättigten Aldehyds und/oder einer ungesättigten Carbonsäure durch Gasphasenoxidation in einem Festbett-Mehrröhrenreaktor. Die teilchenförmigen Katalysatoren weisen ein Loch auf, wobei der Außendurchmesser D1 im Bereich von 3 bis 15 mm liegt und der Durchmesser D2 des Lochs im Bereich von 0,1 x D1 bis 0,7 x D1 liegt. Die mit Katalysator gepackte Schicht jedes Reaktionsrohrs ist in Rohrachsenrichtung in drei Reaktionszonen unterteilt, wobei der Durchmesser des Lochs in der zweiten Reaktionszone kleiner ist als in der ersten Reaktionszone und der Außendurchmesser in der dritten Reaktionszone kleiner ist als in der zweiten Reaktionszone. Die in mm angegebenen Abmessungen (Außendurchmesser (AD) x Höhe (H) x Innendurchmesser (ID)) der Katalysatoren betrugen in den Beispielen 1-3 und 1-5 in der ersten Reaktionszone 7,0 x 7,7 x 3,0, in der zweiten Reaktionszone 6,0 x 6,6 x 2,0 und in der dritten Reaktionszone 5,5 x 6,1 x 2,0.

Die EP1526123B1 beschreibt eine katalytische Gasphasenoxidationsreaktion zur Produktion eines ungesättigten Aldehyds oder einer ungesättigten Carbonsäure in einem Festbett-Mehrröhrenreaktor. Das mit Katalysatoren gepackte Festbett umfasst mindestens zwei Reaktionszonen, wobei das Belegungsvolumen, d.h. das Volumen des Katalysators ohne Berücksichtigung der Hohlvolumina, in einer zur Gasauslassseite nächstliegenden Reaktionszone kleiner als das in einer zur Gaseinlassseite nächstliegenden Reaktionszone ist. In bestimmten Beispielen werden 3 Reaktionszonen mit hohlzylindrischen Katalysatoren eingesetzt. In diesen Beispielen sind die Abmessungen des Katalysators in je zwei unmittelbar benachbarten Reaktionszonen gleich.

In großtechnischen Verfahren sind bereits kleine Ausbeutesteigerungen lohnenswert. Die Aufgabe der vorliegenden Erfindung besteht daher in einer weiteren Steigerung der Ausbeute der Wertprodukte.

Die Aufgabe wird gelöst durch ein Verfahren zur Herstellung eines $\alpha,\beta$-ungesättigten Aldehyds und/oder einer $\alpha,\beta$-ungesättigten Carbonsäure durch Gasphasenoxidation eines Alkens mit molekularem Sauerstoff an einem Katalysatorfestbett, das eine Schüttung hohlzylindrischer Katalysatorformkörper umfasst, deren aktive Masse ein Multimetalloxid ist, dadurch gekennzeichnet, dass

(i) das Katalysatorfestbett mindestens drei aufeinanderfolgende Reaktionszonen umfasst;
(ii) die höchste lokale Temperatur des Katalysatorfestbetts nicht in der dem Reaktorausgang nächsten Reaktionszone auftritt;
(iii) die höchste lokale Temperatur des Katalysatorfestbetts nicht in der dem Reaktoreingang nächsten Reaktionszone auftritt; und
(iv) der Wert WS gemäß der nachstehenden Gleichung in derjenigen Reaktionszone, in der die höchste lokale Temperatur des Katalysatorfestbetts auftritt, kleiner ist als in den anderen Reaktionszonen,

$$WS = \frac{AD\text{-}ID}{2}$$

worin AD für den Außendurchmesser und ID für den Innendurchmesser des Katalysatorformkörpers steht.

Das Alken ist vorzugsweise ausgewählt unter Alkenen mit 3 bis 6, d. h. 3, 4, 5 oder 6 Kohlenstoffatomen; bevorzugt ausgewählt unter Propen und Isobuten. Propen ist besonders bevorzugt. Es kommen insbesondere polymer grade Propen und chemical grade Propen in Betracht, wie es z. B. die DE-A 102 32 748 beschreibt.

Das Verfahren eignet sich besonders zur Herstellung von $\alpha,\beta$-ungesättigten Aldehyden, insbesondere zur Herstellung durch Gasphasenoxidation von Propen und von Methacrolein durch Gasphasenoxidation von Isobuten.

Vorzugsweise handelt es sich um ein Verfahren zur Herstellung von Acrolein durch Gasphasenoxidation von Propen.

Im erfindungsgemäßen Verfahren kommen hohlzylindrische Katalysatorformkörper zum Einsatz, deren aktive Masse ein Multimetalloxid ist. Multimetalloxide zur Gasphasenoxidation von Alkenen zu $\alpha,\beta$-ungesättigten Aldehyden und/oder $\alpha,\beta$-ungesättigten Carbonsäure sind an sich bekannt. Es kommt daher jedes Multimetalloxid in Betracht, das diese Gasphasenoxidation zu katalysieren vermag. Vorzugsweise ist die aktive Masse der Katalysatorformkörper in den Reaktionszonen gleich.

Unter einer Reaktionszone wird ein zusammenhängender Abschnitt der Schüttung verstanden, der Katalysatorformkörper umfasst und in dem die Zusammensetzung der Schüttung im Wesentlichen homogen ist. In der Schüttung können ausschließlich hohlzylindrische Katalysatorformkörper oder auch weitgehend homogene Gemische aus hohlzylindrischen Katalysatorformkörpern und Verdünnungsformkörpern vorliegen. Auch innerhalb einer Reaktionszone ist die Schüttung nur näherungsweise homogen, da die hohlzylindrischen Katalysatorformkörper und ggfs. Verdünnungsformkörper in der Reaktionszone in der Regel zufällig ausgerichtet und statistisch verteilt sind. Die einzelnen Reaktionszonen unterscheiden sich voneinander in mindestens einer Eigenschaft, ausgewählt unter dem Gehalt an inerten Verdünnungsformkörpern, Gestalt der Katalysatoren, Raumerfüllungsgrad der Katalysatoren, Aktivmasseanteil der Katalysatoren und chemischer Zusammensetzung der Aktivmasse.

Über die Katalysatorschüttung stellt sich ein nicht-monotones Temperaturprofil ein. Es kommt in der Katalysatorschüttung zur Ausbildung von lokalen Temperaturmaxima oder sogenannter "Heißer Flecken" (Hot Spots), in denen eine höhere Temperatur herrscht als in benachbarten Teilen der Katalysatorschüttung. Unter der "höchsten lokalen Temperatur des Katalysatorfestbetts" wird das höchste lokale Temperaturmaximum im gesamten Katalysatorfestbett verstanden.

Die Geometrie der hohlzylindrischen Katalysatorformkörper ist durch die Abmessungen Außendurchmesser (AD), Höhe (H) und Innendurchmesser (ID) gekennzeichnet. Die hohlzylindrische Geometrie der vorliegenden Katalysatorformkörper lässt sich durch zwei Zylinder gleicher Höhe beschreiben, deren Achsen zusammenfallen. Einer der Zylinder weist einen Durchmesser ID auf. Der andere Zylinder weist einen Durchmesser AD auf. Die Mantelfläche des inneren Zylinders fällt mit der Innenfläche des hohlzylindrischen Katalysatorformkörpers zusammen. Die Mantelfläche des äußeren Zylinders fällt mit der Außenfläche des hohlzylindrischen Katalysatorformkörpers zusammen.

Der Wert WS entspricht der Wandstärke (WS) der hohlzylindrischen Katalysatorformkörper (Gleichung 1).

$$WS = \frac{AD\text{-}ID}{2} \qquad \text{(Gleichung 1)}$$

Im erfindungsgemäßen Verfahren ist WS in derjenigen Reaktionszone, in der die höchste lokale Temperatur des Katalysatorfestbetts auftritt, kleiner als in den anderen Reaktionszonen.

In wenigstens einer Reaktionszone, vorzugsweise in allen Reaktionszonen, kommen Katalysatorformkörper zum Einsatz, deren geometrisches Volumen vorzugsweise kleiner als 120 mm$^3$, bevorzugt kleiner 80 mm$^3$, besonders bevorzugt kleiner als 60 mm$^3$ ist. Es kann unter Zugrundelegung der Höhe H des Zylinders, des Außendurchmessers AD und des Durchmessers der Innenbohrung ID berechnet werden. Ein Hohlzylinder mit den Abmessungen AD = 4 mm, H = 3 mm und ID = 2 mm besitzt beispielsweise ein geometrisches Volumen von 28,27 mm$^3$.

In wenigstens einer Reaktionszone, vorzugsweise in allen Reaktionszonen, kommen Katalysatorformkörper zum Einsatz, deren Verhältnis von geometrischem Volumen zu geometrischer Oberfläche kleiner 0,9 mm, bevorzugt kleiner 0,7 mm, besonders bevorzugt kleiner 0,55 mm ist. Besonders bevorzugt weisen die Katalysatorformkörper zumindest in der derjenigen Reaktionszone, in der die höchste lokale Temperatur des Katalysatorfestbetts auftritt, ein Verhältnis von geometrischem Volumen zu geometrischer Oberfläche kleiner 0,5 mm, bevorzugt kleiner 0,45 mm, besonders bevorzugt kleiner 0,4 mm auf.

Die geometrische Oberfläche ist eine idealisierte Größe und berücksichtigt nicht die durch die Porosität oder Oberflächenrauhigkeit des Formkörpers bedingte Oberflächenvergrößerung. Die geometrische Oberfläche hohlzylindrischer Katalysatorformkörpers wird durch die folgende Formel berechnet:

$$\frac{\pi}{2}\left((AD)^2\text{-}(ID)^2\right)+\pi(AD+ID)H$$

Vorzugsweise ist die volumenspezifische Katalysatoraktivität derjenigen Reaktionszone, in der die höchste lokale Temperatur des Katalysatorfestbetts auftritt, niedriger als in den anderen Reaktionszonen.

Die (relative) volumenspezifische Katalysatoraktivität kann als auf das Katalysatorschüttungsvolumen bezogene Reaktionsgeschwindigkeit einer einzelnen Reaktionszone ermittelt werden. Die Ermittlung der (relativen) volumenspezifischen Katalysatoraktivität erfolgt unter Verwendung von jeweils nur einer Katalysatorschüttung, die die Zusammensetzung einer Reaktionszone wiederspiegelt. Die übrigen Reaktionsbedingungen, insbesondere die Temperierung des Reaktors, die Zusammensetzung des Reaktionsgases und die Belastung der Schüttung, sowie der Schüttungslänge werden bei der Ermittlung der volumenspezifischen Katalysatoraktivität nicht variiert. Die für die Ermittlung der (relativen) volumenspezifischen Katalysatoraktivität maßgebliche Reaktionsgeschwindigkeit ist die Geschwindigkeit, mit der das Alken umgesetzt wird.

Die volumenspezifische Katalysatoraktivität einer Reaktionszone kann variiert werden, indem man die Katalysatoraktivmassendichte ändert. Die Katalysatoraktivmassendichte ist definiert als das Verhältnis des Gewichts der in der jeweiligen Reaktionszone eingebrachten aktiven Masse zum Volumen der jeweiligen Reaktionszone.

Vorzugsweise ist die Katalysatoraktivmassendichte derjenigen Reaktionszone, in der die höchste lokale Temperatur des Katalysatorfestbetts auftritt, niedriger als in den anderen Reaktionszonen.

Die Katalysatoraktivmassendichte bzw. die volumenspezifische Katalysatoraktivität einer Reaktionszone kann verringert werden, indem man ein weitgehend homogenes Gemisch aus hohlzylindrischen Katalysatorformkörpern und Verdünnungsformkörpern in der Reaktionszone einsetzt. Je höher der Anteil der Verdünnungsformkörper gewählt wird, desto geringer ist der in der Reaktionszone enthaltene Anteil an aktiver Masse.

Die Katalysatoraktivmassendichte bzw. die volumenspezifische Katalysatoraktivität kann verringert werden, indem man hohlzylindrische Katalysatorformkörper einsetzt, in denen die aktive Masse durch feinteilige inerte Verdünnungsmaterialien, wie feinteiliges Siliciumcarbid oder feinteilige Silikate wie Magnesium- und Aluminiumsilikat oder Steatit, verdünnt vorliegt. So kann ein feinteiliges Pulver einer aktiven Masse mit feinteiligem Verdünnungsmaterial vermischt und das dabei resultierende Mischpulver unter Anwendung eines Formgebungsverfahrens (vorzugsweise durch Tablettieren) zu einem geometrischen Formkörper geformt werden.

Die volumenspezifische Katalysatoraktivität kann verringert werden, indem man hohlzylindrische Katalysatorformkörper, die bei erhöhter Temperatur und/oder über einen längeren Zeitraum hinweg kalciniert wurden, in derjenigen Reaktionszone einsetzt, in der die höchste lokale Temperatur des Katalysatorfestbetts auftritt.

Die niedrigere volumenspezifische Katalysatoraktivität wird vorzugsweise zumindest zum Teil, insbesondere bevorzugt ausschließlich durch entsprechende Wahl der Abmessungen der hohlzylindrischen Katalysatorformkörper erreicht.

In einer Ausführungsform ist der Außendurchmesser der Katalysatorformkörper in derjenigen Reaktionszone, in der die höchste lokale Temperatur des Katalysatorfestbetts auftritt, kleiner als der Außendurchmesser der Katalysatorformkörper in den anderen Reaktionszonen und der Innendurchmesser der Katalysatorformkörper in allen Reaktionszonen gleich.

In einer alternativen Ausführungsform ist der Innendurchmesser der Katalysatorformkörper in derjenigen Reaktionszone, in der die höchste lokale Temperatur des Katalysatorfestbetts auftritt, größer als der Innendurchmesser der Katalysatorformkörper in den anderen Reaktionszonen und der Außendurchmesser der Katalysatorformkörper in allen Reaktionszonen gleich.

Vorzugsweise umfasst das Katalysatorfestbett drei aufeinanderfolgende Reaktionszonen, wobei (i) die erste Reaktionszone 70 bis 90 % der volumenspezifischen Katalysatoraktivität der dritten Reaktionszone aufweist, und (ii) die zweite Reaktionszone 50 bis 70 % der volumenspezifischen Katalysatoraktivität der dritten Reaktionszone aufweist.

Vorzugsweise umfasst das Katalysatorfestbett drei aufeinanderfolgende Reaktionszonen, wobei (i) die erste Reaktionszone 70 bis 90 % der Katalysatoraktivmassendichte der dritten Reaktionszone aufweist, und (ii) die zweite Reaktionszone 50 bis 70 % der Katalysatoraktivmassendichte der dritten Reaktionszone aufweist.

Vorteilhafte hohlzylindrische Formkörper weisen ein Verhältnis von Höhe zu Außendurchmesser (H/AD) im Bereich von 0,4 bis 0,9, bevorzugt 0,45 bis 0,85 auf.

Im Allgemeinen weisen die hohlzylindrischen Katalysatorformkörper einen Außendurchmesser von 3 bis 5 mm, eine Höhe von 2 bis 4 mm und einen Innendurchmesser von 1 bis 4 mm auf.

Vorzugsweise ist die Höhe der Katalysatorformkörper in allen Reaktionszonen gleich.

Vorzugsweise umfasst das Katalysatorfestbett drei aufeinanderfolgende Reaktionszonen, wobei (i) die erste Reaktionszone Katalysatorformkörper mit einem Außendurchmesser von 5 mm, einer Höhe von 3 mm und einem Innendurchmesser von 2 mm, (ii) die zweite Reaktionszone Katalysatorformkörper mit einem Außendurchmesser von 5 mm, einer Höhe von 3 mm und einem Innendurchmesser von 3 mm, und (iii) die dritte Reaktionszone Katalysatorformkörper mit einem Außendurchmesser von 5 mm, einer Höhe von 3 mm und einem Innendurchmesser von 2 mm umfasst.

Besonders bevorzugt umfasst das Katalysatorfestbett drei aufeinanderfolgende Reaktionszonen, wobei (i) die erste Reaktionszone Katalysatorformkörper mit einem Außendurchmesser von 5 mm, einer Höhe von 2,5 mm und einem Innendurchmesser von 2 mm, (ii) die zweite Reaktionszone Katalysatorformkörper mit einem Außendurchmesser von 5 mm, einer Höhe von 2,5 mm und einem Innendurchmesser von 3 mm, und (iii) die dritte Reaktionszone Katalysatorformkörper mit einem Außendurchmesser von 5 mm, einer Höhe von 2,5 mm und einem Innendurchmesser von 2 mm umfasst.

Vorzugsweise umfasst das Katalysatorfestbett drei aufeinanderfolgende Reaktionszonen, wobei (i) die erste Reaktionszone Katalysatorformkörper mit einem Außendurchmesser von 5 mm, einer Höhe von 3 mm und einem Innendurchmesser von 2 mm, (ii) die zweite Reaktionszone Katalysatorformkörper mit einem Außendurchmesser von 4 mm, einer Höhe von 3 mm und einem Innendurchmesser von 2 mm, und (iii) die dritte Reaktionszone Katalysatorformkörper mit einem Außendurchmesser von 5 mm, einer Höhe von 3 mm und einem Innendurchmesser von 2 mm umfasst.

Vorzugsweise umfasst das Katalysatorfestbett drei aufeinanderfolgende Reaktionszonen, wobei (i) die erste Reaktionszone Katalysatorformkörper mit einem Außendurchmesser von 5 mm, einer Höhe von 2,5 mm und einem Innendurchmesser von 2 mm, (ii) die zweite Reaktionszone Katalysatorformkörper mit einem Außendurchmesser von 4 mm, einer Höhe von 2,5 mm und einem Innendurchmesser von 2 mm, und (iii) die dritte Reaktionszone Katalysatorformkörper mit einem Außendurchmesser von 5 mm, einer Höhe von 2,5 mm und einem Innendurchmesser von 2 mm umfasst.

Die Stirnflächen der hohlzylindrischen Katalysatorformkörper können auch entweder beide oder nur eine wie in der EP-A 184790 oder der US 4,656,157 beschrieben gekrümmt sein und zwar z. B. so, dass der Radius der Krümmung vorzugsweise das 0,4 bis 5-fache des Außendurchmessers A beträgt. Erfindungsgemäß bevorzugt ist keine der Stirnflächen gekrümmt.

Vorzugsweise umfasst das Katalysatorfestbett drei aufeinanderfolgende Reaktionszonen, wobei (i) die erste Reaktionszone 2 bis 5 % des Volumens des Katalysatorfestbetts, (ii) die zweite Reaktionszone 25 bis 45 % des Volumens des Katalysatorfestbetts, und (iii) die dritte Reaktionszone 50 bis 73 % des Volumens des Katalysatorfestbetts ausmacht.

Als Volumen des Katalysatorfestbetts wird das von der Schüttung eingenommene Leerrohrvolumen verstanden. Es wird berechnet, indem man die Länge des Katalysatorfestbetts mit dessen Querschnittsfläche multipliziert.

Eine oder mehrere Reaktionszonen des Katalysatorfestbetts können Mischungen aus Katalysatorformkörpern und Inertmaterial enthalten. Das Inertmaterial liegt vorzugsweise in Form vor Formkörpern vor. Die Geometrie dieser Verdünnungsformkörper kann im Prinzip beliebig sein. D. h., es können beispielsweise Kugeln, Polygone, Vollzylinder oder auch Hohlzylinder sein. Besonders bevorzugt liegt das Inertmaterial in Form von hohlzylindrischen Formkörpern vor, deren Abmessungen im Wesentlichen dem Außendurchmesser, der Höhe und dem Innendurchmesser der in der jeweiligen Reaktionszone eingesetzten Katalysatorformkörper entsprechen. Vorzugsweise weichen der Außendurchmesser, die Höhe und der Innendurchmesser höchstens um je 0 bis 1 mm vom Außendurchmesser, der Höhe und dem Innendurchmesser der in der jeweiligen Reaktionszone eingesetzten Katalysatorformkörper ab. Vorzugsweise setzt man Steatit als Inertmaterial ein.

Die hohlzylindrischen Katalysatorformkörper bestehen vorzugsweise überwiegend, insbesondere zu 80 bis 100 Gew.-%, weiterhin bevorzugt zu 85 bis 100 Gew.-%, besonders bevorzugt zu 90 bis 100 Gew.-% aus dem Multimetalloxid (sogenannte Vollkatalysatorformkörper). Daneben können Formgebungshilfsmittel und/oder feinteiliges inertes Verdünnungsmaterial in den hohlzylindrischen Katalysatorformkörpern enthalten sein.

Die Formgebungshilfsmittel sind insbesondere Verstärkungsmittel wie Mikrofasern, z.B. aus Glas, Asbest, Siliciumcarbid oder Kaliumtitanat, die sich nach Beendigung der Formgebung förderlich auf den Zusammenhalt des resultierenden Formkörpers auswirken. Andere Formgebungshilfsmittel, insbesondere Gleitmittel, wie z. B. Graphit, Ruß, Polyethylenglykol, Polyacrylsäure, Stearinsäure, Stärke, Mineralöl, Pflanzenöl, Wasser, Glycerin, Celluloseether, Bortrifluorid und/oder Bornitrid, die bei der Herstellung des Katalysatorformkörpers zugesetzt werden können, entweichen insbesondere bei der Kalcination ganz oder teilweise in Form gasförmiger Verbindungen (z. B. $CO$, $CO_2$).

Als feinteiliges inertes Verdünnungsmaterial kommen unter anderen bei hohen Temperaturen gebrannte und dadurch vergleichsweise an Poren arme Elementoxide wie Aluminiumoxid, Siliciumdioxid, Thoriumdioxid und Zirkondioxid in Betracht. Aber auch feinteiliges Siliciumcarbid oder feinteilige Silikate wie Magnesium- und Aluminiumsilikat oder Steatit können als inerte Verdünnungsmaterialien in den Katalysatorformkörpern enthalten sein.

Außerdem kommen als hohlzylindrische Katalysatorformkörper auch hohlzylindrische Schalenkatalysatoren in Betracht. Die hohlzylindrischen Schalenkatalysatoren können beispielsweise eine auf inerte hohlzylindrische Formkörper aufgebrachte aktive Masse enthalten.

Die Dichte der Katalysatorformkörper beträgt vorzugsweise 1,2 bis 2,0 $g/cm^3$. Sie wird berechnet, indem man die Masse des Katalysatorformkörpers durch dessen geometrisches Volumen teilt.

Multimetalloxide zur Gasphasenoxidation von Alkenen zu $\alpha,\beta$-ungesättigten Aldehyden und/oder $\alpha,\beta$-ungesättigten Carbonsäure sind an sich bekannt. Es kommt daher jedes Multimetalloxid in Betracht, das diese Gasphasenoxidation zu katalysieren vermag. Vorzugsweise umfasst das Multimetalloxid mindestens die Elemente Eisen, Bismut und mindestens eines der Elemente Molybdän und Wolfram.

Das Multimetalloxid kann beispielsweise der Formel (I) entsprechen,

$$MO_{12}Bi_aFe_bX^1_cX^2_dX^3_eX^4_fO_n \qquad (I),$$

worin

X$^1$    für Nickel und/oder Kobalt steht,
X$^2$    für Thallium, ein Alkalimetall und/oder ein Erdalkalimetall steht,

X³    für Zink, Phosphor, Arsen, Bor, Antimon, Zinn, Cer, Blei, Vanadium, Chrom und/oder Wolfram steht,

X⁴    für Silicium, Aluminium, Titan und/oder Zirkonium steht,

a     für eine Zahl im Bereich von 0,2 bis 5 steht,

b     für eine Zahl im Bereich von 0,01 bis 10 steht,

c     für eine Zahl im Bereich von 0 bis 10 steht,

d     für eine Zahl im Bereich von 0 bis 2 steht,

e     für eine Zahl im Bereich von 0 bis 8 steht,

f     für eine Zahl im Bereich von 0 bis 10 steht, und

n     für eine Zahl steht, die durch die Wertigkeit und Häufigkeit der von Sauerstoff verschiedenen Elemente in (I) bestimmt wird;

oder der Formel (II) entsprechen,

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (II),$$

worin

Y¹    für Bismut oder für Bismut und mindestens eines der Elemente Tellur, Antimon, Zinn und Kupfer steht,

Y²    für Molybdän oder Wolfram, oder für Molybdän und Wolfram steht,

Y³    für ein Alkalimetall, Thallium und/oder Samarium steht,

Y⁴    für ein Erdalkalimetall, Nickel, Kobalt, Mangan, Zink, Zinn, Cadmium und/oder Quecksilber steht,

Y⁵    für Eisen oder für Eisen und mindestens eines der Elemente Vanadium, Chrom und Cer steht,

Y⁶    für Phosphor, Arsen, Bor, Antimon und/oder Bismut steht,

Y⁷    für ein seltenes Erdmetall, Titan, Zirkonium, Niob, Tantal, Rhenium, Ruthenium, Rhodium, Kupfer, Silber, Gold, Aluminium, Gallium, Indium, Silicium, Germanium, Blei, Thorium und/oder Uran steht,

Y⁸    für Molybdän oder Wolfram, oder für Molybdän und Wolfram steht,

a'    für eine Zahl im Bereich von 0,01 bis 8 steht,

b'    für eine Zahl im Bereich von 0,1 bis 30 steht,

c'    für eine Zahl im Bereich von 0 bis 4 steht,

d'    für eine Zahl im Bereich von 0 bis 20 steht,

e'    für eine Zahl größer 0 im Bereich von 0 bis 20 steht,

f'    für eine Zahl im Bereich von 0 bis 6 steht,

g'    für eine Zahl im Bereich von 0 bis 15 steht,

h'    für eine Zahl im Bereich von 8 bis 16 steht,

x' und y'   für Zahlen stehen, die durch die Wertigkeit und Häufigkeit der von Sauerstoff verschiedenen Elemente in (II) bestimmt werden, und

p und q    für Zahlen stehen, deren Verhältnis p/q 0,1 bis 10 beträgt.

In der Formel (I) betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner steht X¹ vorzugsweise für Kobalt, X² steht vorzugsweise für K, Cs und/oder Sr, besonders bevorzugt für K, X³ steht vorzugsweise für Wolfram, Zink und/oder Phosphor und X⁴ steht vorzugsweise für Si. Besonders bevorzugt weisen die Variablen X¹ bis X⁴ gleichzeitig die vorgenannten Bedeutungen auf.

Besonders bevorzugt weisen alle stöchiometrischen Koeffizienten a bis f und alle Variablen X¹ bis X⁴ gleichzeitig ihre vorgenannten vorteilhaften Bedeutungen auf. Innerhalb der Stöchiometrien der Formel (I) sind jene bevorzugt, die der Formel (Ia) entsprechen

$$MO_{12}Bi_aFe_bX^1_cX^2_dX^4_fO_n \qquad (Ia)$$

worin

X¹    für Co und/oder Ni steht,

X²    für Alkalimetall steht,

X⁴    für Si und/oder Al steht,

a     für eine Zahl im Bereich von 0,3 bis 1 steht,

b für eine Zahl im Bereich von 0,5 bis 10 steht,

c für eine Zahl im Bereich von 2 bis 10 steht,

d für eine Zahl im Bereich von 0 bis 0,5 steht,

f für eine Zahl im Bereich von 0 bis 10 steht, und

n für eine Zahl steht, die durch die Wertigkeit und Häufigkeit der von Sauerstoff verschiedenen Elemente in (Ia) bestimmt wird.

Vorzugsweise liegt in den kristallinen Anteilen des Multimetalloxids der Formel (I) neben $\beta$-$X^1MoO_4$ als Hauptkomponente $Fe_2(MoO_4)_3$ als Nebenkomponente und kein $MoO_3$ vor.

Unter den Metalloxiden der Formel (Ia) haben sich kobalthaltige Multimetalloxide besonders bewährt. In einer besonders bevorzugten Ausführungsform entspricht das Multimetalloxid der Formel (Ia), worin $X^1$ für Co steht, $X^2$ für K steht, $X^4$ für Si steht, a für eine Zahl im Bereich von 0,5 bis 1 steht, b für eine Zahl im Bereich von 1,5 bis 3 steht, c für eine Zahl im Bereich von 7 bis 8,5 steht, d für eine Zahl im Bereich von 0 bis 0,15 steht, f für eine Zahl im Bereich von 0 bis 2,5 steht.

Vorzugsweise erfüllt das Multimetalloxid der Formel (Ia) die folgenden Bedingungen 1, 2 und 3:

Bedingung 1: 12 - c - 1,5·b = A, wobei A für eine Zahl im Bereich von 0,5 bis 1,5 steht;

Bedingung 2: der Quotient a/A steht für eine Zahl im Bereich von 0,2 bis 1,3;

Bedingung 3: der Quotient c/b steht für eine Zahl im Bereich von 2,5 bis 9.

Bevorzugt ist die Zusammensetzung des Multimetalloxids gemäß (Ia) $Mo_{12}Bi_{0,6}Fe_3CO_7K_{0,08}Si_{1,6}O_n$ und besonders bevorzugt $Mo_{12}Bi_{0,6}Fe_{2,1}Co_{8,3}K_{0,08}Si_{1,6}O_n$.

In Multimetalloxiden der Formel (II) sind Bereiche der chemischen Zusammensetzung $[Y^1_{a'}Y^2_{b'}O_{x'}]$ und Bereiche der chemischen Zusammensetzung $[Y^3_{c'}Y^4_{d'}Y^5_{e'}Y^6_{f'}Y^7_{g'}Y^8_{h'}O_{y'}]$ relativ zueinander wie in einem Gemisch aus feinteiligem $[Y^1_{a'}Y^2_{b'}O_{x'}]$ und feinteiligem $[Y^3_{c'}Y^4_{d'}Y^5_{e'}Y^6_{f'}Y^7_{g'}Y^8_{h'}O_{y'}]$ verteilt.

Dabei sind Multimetalloxide der Formel (II) bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$ enthalten, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Ferner ist es von Vorteil, wenn mindestens 25 mol-% (bevorzugt mindestens 50 mol-% und besonders bevorzugt 100 mol-%) des gesamten Anteils $[Y^1_{a'}Y^2_{b'}O_{x'}]_p$ der Multimetalloxide der Stöchiometrie (II) in den Multimetalloxiden der Stöchiometrie (II) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ vorliegen, deren Größtdurchmesser im Bereich 1 nm bis 100 $\mu$m liegt.

Vorteilhafte Multimetalloxide der Stöchiometrie (II) sind solche, in denen $Y^1$ nur für Bismut steht. Innerhalb der Multimetalloxide der Formel (II) sind jene bevorzugt, die der Formel (IIa) entsprechen

$$[Bi_{a'}Y^2_{b'}O_{x'}]_p[Y^3_{c'}Y^4_{d'}Fe_{e'}Y^6_{f'}Y^7_{g'}Y^8_{12}O_{y'}]_q \qquad (IIa),$$

worin

$Y^2$ für Molybdän oder Wolfram, oder für Molybdän und Wolfram steht,

$Y^3$ für ein Alkalimetall und/oder Thallium, vorzugsweise K, Cs, steht,

$Y^4$ für ein Erdalkalimetall, Nickel, Kobalt und/oder Zinn steht,

$Y^6$ für Phosphor, Arsen, Bor, Antimon, und/oder Bismut steht,

$Y^7$ für Titan, Zirkonium, Aluminium, Silicium, Kupfer, Silber und/oder Gold, vorzugsweise Si steht,

$Y^8$ für Molybdän oder Wolfram, oder für Molybdän und Wolfram steht

a' für eine Zahl im Bereich von 0,1 bis 2 steht,

b' für eine Zahl im Bereich von 0,2 bis 4 steht,

c' für eine Zahl im Bereich von 0,02 bis 2 steht,

d' für eine Zahl im Bereich von 3 bis 10 steht,

e' für eine Zahl im Bereich von 0,01 bis 5, vorzugsweise 0,1 bis 4 steht,

f' für eine Zahl im Bereich von 0 bis 5 steht,

g' für eine Zahl im Bereich von 0 bis 10, vorzugsweise für eine Zahl größer 0 im Bereich von 0 bis 10, besonders bevorzugt für eine Zahl im Bereich von 0,2 bis 10 und ganz besonders bevorzugt für eine Zahl im Bereich von 0,4 bis 3 steht,

x' und y' für Zahlen stehen, die durch die Wertigkeit und Häufigkeit der von Sauerstoff verschiedenen Elemente in (IIa) bestimmt werden, und

p und q     für Zahlen stehen, deren Verhältnis p/q 0,1 bis 5, vorzugsweise 0,4 bis 2 beträgt.

Innerhalb der Multimetalloxide der Formel (IIa) sind diejenigen bevorzugt, in denen $Y^2$ für Wolfram und $Y^8$ für Molybdän steht.

Ferner sind Multimetalloxide der Stöchiometrie (IIa) bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Bi_aY^2_{b'}O_{x'}$ enthalten, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Entsprechend ist es von Vorteil, wenn mindestens 25 mol-% (bevorzugt mindestens 50 mol-% und besonders bevorzugt 100 mol-%) des gesamten Anteils $[Bi_aY^2_{b'}O_{x'}]_p$ der Multimetalloxide der Stöchiometrie (IIa) in den Multimetalloxiden der Stöchiometrie (IIa) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $[Bi_aY^2_{b'}O_{x'}]$ vorliegen, deren Größtdurchmesser im Bereich 1 nm bis 100 $\mu$m liegt.

Bevorzugt ist die Zusammensetzung des Multimetalloxids gemäß (IIa) $[Bi_1W_2O_{x'}]_{0,5}[K_{0,08}Co_{5,5}Fe_3Si_{1,6}Mo_{12}O_{y'}]$ und besonders bevorzugt $[Bi_1W_2O_{x'}]_{0,4}[K_{0,08}Co_{5,5}Fe_3Si_{1,6}Mo_{12}O_{y'}]$.

Vorzugsweise zeichnet sich die das Multimetalloxid dadurch aus, dass es im Wesentlichen keine lokalen Zentren aus Elementoxiden (z. B. Eisenoxid) aufweist. Vielmehr sind diese Elemente weitestgehend Bestandteil komplexer, gemischter Oxomolybdate. Dies verringert die unerwünschte Vollverbrennung organischer Reaktionsgasbestandteile.

Das Multimetalloxid kann dadurch hergestellt werden, dass man von geeigneten Quellen ihrer (insbesondere der von Sauerstoff verschiedenen) elementaren Konstituenten ein vorzugsweise feinteiliges, der jeweiligen Stöchiometrie entsprechend zusammengesetztes, inniges Trockengemisch erzeugt und dieses, wahlweise nach erfolgter Formgebung zu hohlzylindrischen Formkörpern, die wahlweise unter Mitverwendung von Formgebungshilfsmitteln erfolgt, bei Temperaturen im Bereich von 350 bis 650 °C kalciniert. Mit dem Begriff Quelle ist in dieser Schrift ein Ausgangsstoff zur Herstellung des Multimetalloxids bezeichnet. Die Kalcination kann sowohl unter Inertgas als auch unter einer oxidativen Atmosphäre wie z. B. Luft (oder ein anderes Gemisch aus Inertgas und molekularem Sauerstoff) sowie auch unter reduzierender Atmosphäre (z. B. ein Gemisch aus Inertgas, $NH_3$, CO und/oder $H_2$) erfolgen oder unter Vakuum. Die Kalcinationsdauer kann einige Minuten bis einige Stunden betragen und nimmt üblicherweise mit der Kalcinationstemperatur ab.

Als Quellen kommen solche Verbindungen in Betracht, bei denen es sich bereits um Oxide von im Metalloxid enthaltenen Metallen handelt und/oder solche Verbindungen, die durch Erhitzen, mindestens in Anwesenheit von Sauerstoff, in Oxide überführbar sind.

Neben den Oxiden kommen als Quellen vor allem Halogenide, Nitrate, Formiate, Oxalate, Citrate, Acetate, Carbonate, Aminkomplexe, Ammoniumsalze und/oder Hydroxide von im Metalloxid enthaltenen Metallen sowie Hydrate der vorgenannten Salze in Betracht. Verbindungen wie $NH_4OH$, $(NH_4)CO_3$, $NH_4NO_3$, $NH_4CHO_2$, $CH_3COOH$, $NH_4CH_3CO_2$ und/oder Ammoniumoxalat, die spätestens beim späteren Kalcinieren zu gasförmig entweichenden Verbindungen zerfallen und/oder zersetzt werden können, können in das innige Trockengemisch zusätzlich eingearbeitet werden. Als solche sich beim Kalcinieren zersetzende Substanzen kommen auch organische Materialien, wie z. B. Stearinsäure, Malonsäure, Ammoniumsalze der vorgenannten Säuren, Stärken (z. B. Kartoffelstärke und Maisstärke), Cellulose, gemahlene Nussschalen und feinteiliges Kunststoffmehl (z. B. aus Polyethylen, Polypropylen etc.), in Betracht.

Das innige Vermischen der Quellen zur Herstellung des Multimetalloxids kann in trockener oder in nasser Form erfolgen. Erfolgt es in trockener Form, so werden die Quellen zweckmäßigerweise als feinteilige Pulver eingesetzt. Vorzugsweise erfolgt das innige Vermischen jedoch in nasser Form.

Erfindungsgemäß vorteilhaft werden dabei die Quellen in Form von Lösungen und/oder Suspensionen miteinander vermischt und die dabei resultierende nasse Mischung anschließend zum innigen Trockengemisch getrocknet. Als Lösungs- und/oder Suspendiermittel wird bevorzugt Wasser bzw. eine wässrige Lösung eingesetzt.

Besonders innige Trockengemische werden beim vorstehend beschriebenen Mischverfahren dann erhalten, wenn ausschließlich von in gelöster Form vorliegenden Quellen und/oder von kolloidal gelösten Quellen der elementaren Konstituenten ausgegangen wird. Generell kann eine Ausgangsverbindung Quelle für nur einen oder für mehr als einen elementaren Konstituenten sein. In dementsprechender Weise kann eine vorstehend aufgeführte in gelöster Form bzw. kolloidal vorliegende Quelle nur einen oder auch mehr als einen elementaren Konstituenten aufweisen. Die Trocknung der resultierenden nassen Mischungen erfolgt vorzugsweise durch Sprühtrocknung.

Das Element Silizium kann beispielsweise in Form eines Kieselsols zur Herstellung der nassen Mischung eingebracht werden. Kieselsole sind kolloidale Lösungen von amorphem Siliciumdioxid in Wasser. Sie sind wasserflüssig und enthalten keine sedimentierbaren Bestandteile. Ihr $SiO_2$-Gehalt kann bei oft jahrelanger Haltbarkeit (ohne Sedimentation) bis zu 50 Gew.-% und mehr betragen.

Eine Quelle kann auch teilweise gelöst sowie teilweise kolloidal vorliegen.

Eine günstige Mo-Quelle ist Ammoniumheptamolybdattetrahydrat. Weitere mögliche Mo-Quellen sind Ammoniumorthomolybdat ($(NH_4)_2MoO_4$), Ammoniumdimolybdat ($(NH_4)_2Mo_2O_7$), Ammoniumtetramolybdatdihydrat ($(NH_4)_2Mo_4O_{13}$ x $5H_2O$) und Ammoniumdecamolybdatdihydrat ($(NH_4)_4Mo_{10}O_{32}$ x $2\,H_2O$). Grundsätzlich ist aber auch z. B. Molybdäntrioxid einsetzbar.

Bevorzugte K-Quelle ist KOH (Kaliumhydroxid). Grundsätzlich kann aber auch $KNO_3$ bzw. dessen Hydrat als K-Quelle verwendet werden.

Bevorzugte Bi-Quellen weisen das Bi als $Bi^{3+}$ auf. Als Bi-Quellen kommen z. B. Bismut(III)oxid, Bismut(III)oxidnitrat (Bismutsubnitrat), Bismut(III)halogenid (z. B. Fluorid, Chlorid, Bromid, Iodid) und insbesondere Bismut(III)nitratpentahydrat in Betracht. Besonders bevorzugt werden salpetersaure wässrige Lösungen von Bismutnitrat eingesetzt.

Bevorzugte Fe-Quellen sind Salze des $Fe^{3+}$, unter denen Eisen(III)nitrathydrate besonders bevorzugt sind (vgl. z. B. DE-A 102007003076). Besonders bevorzugte Fe-Quelle ist Eisen(III)nitratnonahydrat. Selbstverständlich können auch Salze des $Fe^{2+}$ als Fe-Quelle verwendet werden.

Zur Herstellung des Multimetalloxids wird, bezogen auf die in ihnen enthaltene molare Gesamtmenge an Fe, mindestens 50 mol-%, besser mindestens 75 mol-% und bevorzugt mindestens 95 mol-% in Form einer Fe-Quelle eingebracht, die das Fe als $Fe^{3+}$ aufweist. Auch können Fe-Quellen verwendet werden, die sowohl $Fe^{2+}$ wie auch $Fe^{3+}$ aufweisen.

Geeignete Co-Quellen sind dessen Salze, die das Co als $Co^{2+}$ und/oder $Co^{3+}$ aufweisen. Als solche seien beispielhaft das Kobalt(II)nitrathexahydrat, das $Co_3O_4$, das CoO, das Kobalt(II)formiat und das Kobalt(III)nitrat genannt. Kobalt(II)nitrathexahydrat ist bevorzugt. Besonders bevorzugt werden salpetersaure wässrige Lösungen von Cobaltnitrat eingesetzt.

Im Allgemeinen erfolgt die Herstellung der nassen Mischung bevorzugt an Luft (vorteilhaft ist die nasse Mischung an Luft gesättigt). Dies gilt insbesondere dann, wenn als Co-Quelle und Fe-Quelle Salze des $Co^{2+}$ sowie Salze des $Fe^{2+}$ verwendet werden. Vor allem dann, wenn es sich bei diesen Salzen um die Nitrate und/oder deren Hydrate handelt.

Vorzugsweise erfolgt das innige Vermischen der Quellen zur Herstellung des Multimetalloxids in nasser Form, besonders bevorzugt in wässriger Form. Hierbei wird vorzugsweise aus mindestens einer Quelle der Elemente Co, Fe und Bi eine wässrige Lösung A hergestellt. Bevorzugt ist die wässrige Lösung A eine wässrige Lösung der Nitrate bzw. Nitrat-Hydrate von Co, Bi und Fe. Besonders bevorzugt ist die wässrige Lösung A eine wässrige Lösung der Nitrate bzw. Nitrat-Hydrate in wässriger Salpetersäure. Eine solche Lösung kann auch durch Lösen der entsprechenden Metalle in wässriger Salpetersäure erhalten werden.

Aus mindestens einer Quelle des Elements Mo sowie wahlweise einer oder mehrerer Quellen des Elements K wird eine wässrige Lösung B hergestellt.

Bevorzugte Mo-Quelle zur Zubereitung einer wässrigen Lösung B ist Ammoniumheptamolybdat-tetrahydrat ($(NH_4)_6Mo_7O_{24}$ x $4H_2O$). Enthält die wässrige Lösung B K, so wird als dessen Quelle zur Zubereitung der wässrigen Lösung B vorteilhaft KOH verwendet.

Der Gesamtgehalt der wässrigen Lösung A an Co, Fe und Bi beträgt zweckmäßig, bezogen auf die Menge des in der wässrigen Lösung A enthaltenen Wassers, 10 bis 25 Gew.-%, vorteilhaft 15 bis 20 Gew.-%.

Der Gesamtgehalt der wässrigen Lösung B an Mo beträgt zweckmäßig, bezogen auf die Menge des in der wässrigen Lösung B enthaltenen Wassers, 3 bis 25 Gew.-%, vorteilhaft 5 bis 15 Gew.-%.

Vorzugsweise werden die wässrige Lösung A und die wässrige Lösung B miteinander vermischt. Vorteilhaft wird dabei so verfahren, dass die wässrige Lösung A kontinuierlich in die wässrige Lösung B eingerührt wird, wobei die vorgelegte wässrige Lösung B dabei vorzugsweise intensiv gerührt wird. Der Gesamtgehalt der resultierenden nassen Mischung aus wässriger Lösung A und wässriger Lösung B an Mo, Co, Fe und Bi beträgt zweckmäßig, bezogen auf die Menge des in der nassen Mischung enthaltenen Wassers, 5 bis 25 Gew.-%, vorteilhaft 8 bis 20 Gew.-%.

Insbesondere zur Herstellung von Aktivmassen der Stöchiometrie der allgemeinen Formel II oder IIa ist es vorteilhaft, ein Mischoxid $Y^1_{a'}Y^2_{b'}O_{x'}$ bzw. $Bi_{a'}Y^2_{b'}O_{x'}$ als Quelle der Elemente $Y^1$, $Y^2$ bzw. Bi, $Y^2$ in Abwesenheit der übrigen Konstituenten des Multimetalloxids vorzubilden. Nach der Vorbildung wird das Mischoxid mit Quellen der übrigen Konstituenten des Multimetalloxids vereinigt und daraus das innige Trockengemisch erzeugt. Kommt das Mischoxid bei der Herstellung des innigen Trockengemischs mit Lösungsmittel, insbesondere mit Wasser in Kontakt, so wird vorzugsweise darauf geachtet, dass das Mischoxid $Y^1_{a'}Y^2_{b'}O_{x'}$ bzw. $Bi_{a'}Y^2_{b'}O_{x'}$ nicht in nennenswertem Umfang in Lösung geht. Ausführlich wird eine wie vorstehend beschriebene Herstellweise in den Schriften DE-A 4407020, EP-A 835, EP-A 575897 und DE-C 3338380 beschrieben.

Enthält das Multimetalloxid den elementaren Konstituenten Si, so wird als Quelle desselben vorteilhaft wässriges Kieselsol (vgl. z. B. DE-A 102006044520) verwendet und dieses in zweckmäßiger Weise in die nasse Mischung eingerührt, wobei der nassen Mischung vorab Wasser zugesetzt werden kann. Wässriges Kieselsol und Wasser werden vorzugsweise gleichzeitig zugesetzt.

Bei der Sprühtrocknung der nassen Mischung wird diese zunächst in feinteilige Tröpfchen zerteilt und die feinteiligen Tröpfchen anschließend getrocknet. Bevorzugt erfolgt die Sprühtrocknung im Heißluftstrom. Grundsätzlich können zur Sprühtrocknung aber auch andere heiße Gase verwendet werden (z. B. Stickstoff oder mit Stickstoff verdünnte Luft sowie sonstige inerte Gase).

Die Sprühtrocknung kann dabei sowohl im Gleichstrom als auch im Gegenstrom der Tröpfchen zum heißen Gas erfolgen. Typische Gaseintrittstemperaturen liegen dabei im Bereich von 250 bis 450 °C, vorzugsweise 270 bis 370 °C. Typische Gasaustrittstemperaturen liegen im Bereich von 100 bis 160 °C. Vorzugsweise erfolgt die Sprühtrocknung im Gleichstrom der Tröpfchen zum heißen Gas.

Die nasse Mischung kann auch durch konventionelles Eindampfen (vorzugsweise bei vermindertem Druck; die Trocknungstemperatur wird im Regelfall 150 °C nicht überschreiten) getrocknet werden. Grundsätzlich kann die Trocknung der nassen Mischung auch durch Gefriertrocknung erfolgen.

Durch Sprühtrocknung erhaltenes inniges Trockengemisch wird im Folgenden auch als Sprühpulver bezeichnet.

Grundsätzlich kann das innige Trockengemisch als solches kalciniert werden. Häufig ist das Sprühpulver jedoch für eine unmittelbare Kalcination zu feinteilig.

Das Sprühpulver kann durch nachfolgendes Kompaktieren vergröbert werden (in der Regel auf eine Körnung von 100 μm bis 1 mm). Anschließend kann mit dem vergröberten Pulver die Formung des hohlzylindrischen Formkörpers erfolgen, wobei bei Bedarf zuvor nochmals feinteiliges Gleitmittel zugegeben werden kann. Eine solche Kompaktierung zum Zweck der Kornvergröberung kann beispielsweise mittels eines Kompaktierers der Fa. Hosokawa Bepex GmbH (D-74211 Leingarten), vom Typ Kompaktor K 200/100 erfolgen.

Um eine hohe mechanische Stabilität des hohlzylindrischen Katalysatorformkörpers zu gewährleisten, steuert man die Kompaktierung so, dass die erhaltenen vergröberten Pulverpartikel eine Schüttdichte aufweisen, die die angestrebte Dichte des geformten, nicht kalcinierten hohlzylindrischen Formkörpers deutlich unterschreitet. D.h. bei der Formgebung, z.B. durch Tablettierung, erfolgt eine weitergehende Verdichtung der vergröberten Pulverpartikel.

Erfolgt die Kompaktierung trocken, kann vor der Kompaktierung z. B. feinteiliger Graphit und/oder andere in dieser Schrift genannte Formgebungshilfsmittel (z. B. Gleitmittel und/oder Verstärkungsmittel) unter das Sprühpulver gemischt werden (z. B. mit einem Röhnradmischer). Beispielsweise kann die Kompaktierung mit einem zwei gegenläufige Stahlwalzen aufweisenden Kalander durchgeführt werden. Anschließend kann das Kompaktat zielgerichtet auf die für die beabsichtigte Weiterverwendung angemessene Partikelgröße zu einem feinteiligen Vorläufermaterial zerkleinert werden. Dies kann z. B. dadurch erfolgen, dass das Kompaktat durch ein Sieb mit definierter Maschenweite gedrückt wird.

Die Kompaktierung kann grundsätzlich auch feucht erfolgen. Beispielsweise kann das Sprühpulver unter Zusatz von Wasser geknetet werden. Im Anschluss an die Knetung kann die geknetete Masse auf die Folgeverwendung abgestimmt wieder zur gewünschten Feinteiligkeit zerkleinert (vgl. z. B. DE-A 10049873) und zu einem feinteiligen Vorläufermaterial getrocknet werden.

Die wie beschrieben erhältlichen feinteiligen Vorläufermaterialien können nun als solche kalciniert oder zunächst zu hohlzylindrischen Formkörpern geformt und anschließend kalciniert werden.

Wird das feinteilige Vorläufermaterial als solches kalciniert, kann anschließend ein hohlzylindrischer Katalysatorformkörper durch Formgebung zur hohlzylindrischen Form (z. B. durch Tablettieren, Extrudieren oder Strangpressen) hergestellt werden, wobei gegebenenfalls Formgebungshilfsmittel, wie Gleitmittel und/oder Verstärkungsmittel zugesetzt werden können. Bevorzugte Gleitmittel sind Graphit oder Stearinsäure. Bevorzugte Verstärkungsmittel sind Mikrofasern aus Glas, Asbest, Siliciumcarbid oder Kaliumtitanat.

Vorzugsweise wird das innige Trockengemisch, bevorzugt das Sprühpulver, durch Verdichten zu hohlzylindrischen Vorläuferformkörpern geformt und die hohlzylindrischen Vorläuferformkörper durch Kalcination in die hohlzylindrischen Katalysatorformkörper überführt.

Diese Vorgehensweise ist insbesondere dann bevorzugt, wenn das innige Vermischen der Quellen der elementaren Konstituenten des Multimetalloxids zum feinteiligen innigen Trockengemisch in trockener Form erfolgt (vgl. z. B. WO 2008/087116 und DE-A 102008042060).

Als Formgebungshilfsmittel können beispielsweise Gleitmittel wie z. B. Graphit, Ruß, Polyethylenglykol, Polyacrylsäure, Stearinsäure, Stärke, Mineralöl, Pflanzenöl, Wasser, Glycerin, Celluloseether, Bortrifluorid und/oder Bornitrid zugegeben werden. Ferner kommen als Formgebungshilfsmittel Verstärkungsmittel wie Mikrofasern aus Glas, Asbest, Siliciumcarbid oder Kaliumtitanat in Betracht, die sich nach Beendigung der Formgebung durch Verdichten förderlich auf den Zusammenhalt des resultierenden Formkörpers auswirken. Eine Mitverwendung von Gleitmitteln im Rahmen einer entsprechenden Formgebung findet sich z. B. in den Schriften DE-A 102007004961, WO 2008/087116, WO 2005/030393, US-A 2005/0131253, WO 2007/017431, DE-A 102007005606 und in der DE-A 102008040093.

Vorzugsweise wird ausschließlich feinteiliger Graphit als Gleitmittel verwendet. Dabei kommen als zu verwendende feinteilige Graphite insbesondere jene in Betracht, die in den Schriften WO 2005/030393, US-A 2005/0131253, WO 2008/087116 und DE-A 102007005606 empfohlen werden. Dies gilt insbesondere für jene Graphite, die in diesen Schriften in den Beispielen und Vergleichsbeispielen eingesetzt werden. Ganz besonders bevorzugte Graphite sind Asbury 3160 und Asbury 4012 der Firma Asbury Graphite Mills, Inc., New Jersey 08802, USA und Timrex® T44 der Firma Timcal Ltd., 6743 Bodio, Schweiz.

Bezogen auf das Gewicht des innigen Trockengemischs kann diese z. B. bis zu 15 Gew.-% an feinteiligem Gleitmittel (z. B. Graphit) enthalten. Meist liegt der Gleitmittelgehalt in jedoch bei höchstens 9 Gew.-%, vielfach bei höchstens 5 Gew.-%, oft bei höchstens 4 Gew.-%; dies insbesondere dann, wenn das feinteilige Gleitmittel Graphit ist. In der Regel beträgt die vorgenannte Zusatzmenge zumindest 0,5 Gew.-%, meist zumindest 2,5 Gew.-%.

Im Allgemeinen erfolgt die Formgebung zum hohlzylindrischen Vorläuferformkörper durch Einwirkung äußerer Kräfte (Druck) auf das Trockengemisch. Der dabei anzuwendende Formgebungsapparat bzw. die dabei anzuwendende Formgebungsmethode unterliegt keiner Beschränkung.

Beispielsweise kann die Formgebung durch Strangpressen, Tablettieren oder Extrudieren erfolgen. Dabei wird das innige Trockengemisch vorzugsweise anfasstrocken eingesetzt. Es kann jedoch z. B. bis zu 10% seines Gesamtgewichts an Substanzen enthalten, die bei Normalbedingungen (25 °C, 1 atm (1,01 bar)) flüssig sind. Auch kann das innige Trockengemisch feste Solvate (z. B. Hydrate) enthalten, die solche flüssigen Substanzen in chemisch und/oder physikalisch gebundener Form aufweisen. Das innige Trockengemisch kann auch völlig frei von solchen Substanzen sein.

Bevorzugtes Formgebungsverfahren ist die Tablettierung. Die Grundzüge des Tablettierens sind z. B. in "Die Tablette", Handbuch der Entwicklung, Herstellung und Qualitätssicherung, W. A. Ritschel und A. Bauer-Brandl, 2. Auflage, Edition Verlag Aulendorf, 2002 beschrieben und auf die Tablettierung des innigen Trockengemischs übertragbar.

Für die Formgebung zum hohlzylindrischen Katalysatorvorläuferformkörper kommt z.B. ein Kilian Rundläufer (der Fa. Kilian in D-50735 Köln) vom Typ RX 73 oder S 100 in Betracht. Alternativ kann eine Tablettenpresse der Fa. Korsch (D-13509 Berlin) vom Typ PH 800-65 eingesetzt werden.

Bei der Tablettierung beträgt die die Tablettiermaschine umgebende Temperatur normalerweise 25 °C. Anwendungstechnisch zweckmäßig liegen die Partikeldurchmesser des innigen Trockengemischs, wahlweise als Ergebnis einer Vorvergröberung durch Kompaktieren, im Bereich 100 bis 2000 $\mu$m, bevorzugt 150 bis 1500 $\mu$m, besonders bevorzugt 400 bis 1250 $\mu$m, oder 400 bis 1000 $\mu$m, oder 400 bis 800 $\mu$m (vorab der Verdichtung eingemischtes Formgebungshilfsmittel ist dabei nicht mitberücksichtigt). Die Formgebungsdrucke betragen vorteilhaft 50 bis 5000 kg/cm$^2$, bevorzugt 200 bis 3500 kg/cm$^2$, besonders bevorzugt 600 bis 2500 kg/cm$^2$.

Vorzugsweise weisen die hohlzylindrischen Vorläuferformkörper eine möglichst geringe Restfeuchtigkeit auf.

Vorzugsweise liegt der Restfeuchtegehalt der hohlzylindrischen Vorläuferformkörper bei höchstens 10 Gew.-%, besser höchstens 8 Gew.-%, noch besser höchstens 6 Gew.-%, und am Besten höchstens 4 Gew.-% oder höchstens 2 Gew.-% (die Restfeuchtebestimmung kann dabei wie in "Die Bibliothek der Technik", Band 229, "Thermogravimetrische Materialfeuchtebestimmung", Grundlagen und praktische Anwendungen, Horst Nagel, Verlag moderne Industrie, beschrieben erfolgen (z. B. mit Hilfe eines Computrac MAX 5000 XL von Arizona Instruments)).

Vor diesem Hintergrund sollte bereits eine Sprühtrocknung der nassen Mischung so durchgeführt werden, dass das resultierende Sprühpulver einen möglichst geringen Restfeuchtegehalt aufweist.

Hohlzylindrische Vorläuferformkörper sollten möglichst unter Ausschluss von (Luftfeuchtigkeit aufweisender) Umgebungsluft gelagert werden (vorzugsweise erfolgt die Lagerung bis zur Kalcination unter wasserfreiem Inertgas bzw. unter vorab getrockneter Luft).

Vorteilhaft wird bereits die Formgebung des innigen Trockengemisches unter Ausschluss von (Luftfeuchtigkeit aufweisender) Umgebungsluft (z. B. unter N$_2$-Atmosphäre) durchgeführt.

Die Kalcination der hohlzylindrischen Formkörper (bzw. generell von nicht kalcinierten feinteiligen Vorläufermaterial oder von mit diesem beschichteten Trägerformkörpern) erfolgt normalerweise bei Temperaturen, die mindestens 350 °C erreichen oder in der Regel überschreiten. Normalerweise wird im Rahmen der Kalcination die Temperatur von 650 °C jedoch nicht überschritten (der Begriff der Kalcinationstemperatur meint dabei in dieser Schrift die im Kalcinationsgut vorliegende Temperatur). Vorzugsweise wird im Rahmen der Kalcination die Temperatur von 600 °C, bevorzugt die Temperatur von 550 °C und häufig die Temperatur von 500 °C nicht überschritten. Ferner wird im Rahmen der vorstehenden Kalcination vorzugsweise die Temperatur von 380 °C, mit Vorteil die Temperatur von 400 °C, mit besonderem Vorteil die Temperatur von 420 °C und ganz besonders bevorzugt die Temperatur von 440 °C überschritten.

Die Kalcination kann in ihrem zeitlichen Ablauf auch in mehrere Abschnitte gegliedert sein. Bevorzugte Temperaturfenster für die Endkalcinationstemperatur liegen im Temperaturbereich von 400 °C bis 600 °C, vorzugsweise 420 bis 550 °C, besonders bevorzugt 440 bis 500 °C.

Die Dauer der Kalcination beträgt in der Regel mehr als 10 h. Meist wird die Dauer 45 h, bzw. 25 h nicht überschritten. Oft liegt die Dauer unterhalb von 20 h. Grundsätzlich erfolgt bei höheren Kalcinationstemperaturen in der Regel eine kürzere Kalcination als bei niedrigeren Kalcinationstemperaturen.

Vorzugsweise erstreckt sich die Kalcinationsdauer im Temperaturbereich von 430 °C bis 500 °C auf 10 bis 20 h.

Vorzugsweise erfolgt vor der Kalcination eine oft stufenweise thermische Vorbehandlung bei Temperaturen im Bereich von 110 °C bis 350 °C, vorzugsweise 120 °C bis 320 °C, besonders bevorzugt 130 °C bis 290 °C. Eine solche thermische Vorbehandlung wird zweckmäßig so lange durchgeführt, bis die sich unter den Bedingungen der thermischen Vorbehandlung zu gasförmigen Verbindungen zersetzenden Bestandteile weitgehend (vorzugsweise vollständig) zu gasförmigen Verbindungen zersetzt worden sind (der diesbezüglich erforderliche Zeitaufwand kann z. B. 3 h bis 10 h, häufig 4 h bis 8 h betragen.

Die Dauer und der Temperaturbereich der thermischen Vorbehandlung wird dabei vorzugsweise so gewählt, dass die sich dabei einstellende maximale und auf die Masse des Katalysatorvorläuferformkörpers bezogene relative Massenänderung einen Wert von 1% pro Minute nicht überschreitet, um eine starke Beeinträchtigung der mechanischen Katalysatorformkörperstabilität durch z.B. Rissbildung durch die im Katalysatorvorläuferformkörper entstehenden Zersetzungsgase sicher zu vermeiden.

Sowohl die Kalcination, als auch die vor der Kalcination erfolgende thermische Vorbehandlung kann sowohl unter Inertgas als auch unter einer oxidativen Atmosphäre wie z. B. Luft (oder eines Gemisch aus Inertgas und molekularem Sauerstoff) sowie unter reduzierender Atmosphäre (z. B. ein Gemisch aus Inertgas, $NH_3$, CO und/oder $H_2$ oder unter Methan, Acrolein, Methacrolein) erfolgen. Die Kalcination und/oder die thermische Behandlung können auch unter Vakuum ausgeführt werden. Auch kann die Atmosphäre über den Verlauf der Kalcination und/oder der thermischen Vorbehandlung hinweg variiert werden.

Vorzugsweise erfolgt die Kalcination und wahlweise auch die vor der Kalcination erfolgende thermische Vorbehandlung in einer oxidierenden Atmosphäre. Zweckmäßig besteht diese überwiegend aus stehender oder (bevorzugt) bewegter Luft (besonders bevorzugt wird das Kalcinationsgut von einem Luftstrom durchströmt). Die oxidierende Atmosphäre kann jedoch ebenso aus einem stehenden oder bewegten Gemisch aus z. B. 25 Vol.-% $N_2$ und 75 Vol.-% Luft, oder 50 Vol.-% $N_2$ und 50 Vol.-% Luft, oder 75 Vol.-% $N_2$ und 25 Vol.-% Luft bestehen (eine Atmosphäre aus 100 Vol.-% $N_2$ ist ebenfalls möglich).

Prinzipiell kann die Kalcination und wahlweise auch die vor der Kalcination erfolgende thermische Vorbehandlung in den unterschiedlichsten Ofentypen wie z. B. beheizbare Umluftkammern (Umluftöfen, z. B. Umluftschachtöfen), Hordenöfen, Drehrohröfen, Bandkalcinierern oder Schachtöfen durchgeführt werden. Vorzugsweise verwendet man eine Bandkalciniervorrichtung, wie sie die DE-A 10046957 und die WO 02/24620 empfehlen. Eine Heißpunktausbildung innerhalb des Kalcinationsgutes wird dabei weitestgehend dadurch vermieden, dass mit Hilfe von Ventilatoren durch ein das Kalcinationsgut tragendes gasdurchlässiges Förderband erhöhte Volumenströme an Kalcinationsatmosphäre durch das Kalcinationsgut gefördert werden.

Bei der Kalcination und gegebenenfalls auch bei der vor der Kalcination erfolgenden thermischen Vorbehandlung können Formgebungshilfsmittel erhalten bleiben oder zu entweichenden gasförmigen Verbindungen (z. B. CO, $CO_2$) umgesetzt werden.

Man kann das kalcinierte Multimetalloxid zu einem feinteiligen Pulver mahlen, dieses mit feinteiligem inertem Verdünnungsmaterial vermischen und das so erhaltene Mischpulver unter Anwendung eines in dieser Schrift vorgestellten Formgebungsverfahrens (vorzugsweise durch Tablettieren) zu einem hohlzylindrischen Formkörper formen. Durch nachfolgendes nochmaliges Kalcinieren diese Formkörpers wird dann der hohlzylindrischen Katalysatorformkörper erhalten.

Das feinteilige inerte Verdünnungsmaterial kann alternativ auch in die nasse Mischung vor deren Trocknung eingearbeitet werden. Des Weiteren kann eine Einarbeitung von feinteiligem inertem Verdünnungsmaterial in ein feinteiliges Trockengemisch von Quellen der elementaren Konstituenten des Multimetalloxids erfolgen. Solche Vorgehensweisen sind jedoch weniger bevorzugt.

Die spezifische Oberfläche der hohlzylindrischen Katalysatorformkörper beträgt vorteilhaft 2 bis 20 bzw. 15 $m^2$/g, vorzugsweise 3 bis 10 $m^2$/g und besonders bevorzugt 4 bis 8 $m^2$/g. Das Porengesamtvolumen liegt vorteilhaft im Bereich von 0,1 bis 1 $cm^3$/g bzw. bis 0,8 $cm^3$/g, vorzugsweise im Bereich 0,1 bis 0,5 $cm^3$/g und besonders bevorzugt im Bereich 0,2 bis 0,4 $cm^3$/g.

Alle Angaben in dieser Schrift zu spezifischen Oberflächen von Feststoffen beziehen sich auf Bestimmungen nach DIN 66131 (Bestimmung der spezifischen Oberfläche von Feststoffen durch Gasadsorption ($N_2$) nach Brunauer-Emmert-Teller (BET)), soweit nicht ausdrücklich etwas anderes erwähnt wird.

Vorzugsweise tragen Poren mit einem Porenradius von höchstens 0,1 $\mu$m zum Porengesamtvolumen höchstens 0,05 $cm^3$/g bei. Wenn der Beitrag solcher vergleichsweise enger Poren zum Gesamtporenvolumen mehr als 0,05 $cm^3$/g beträgt, kann durch eine Erhöhung der Kalcinationsdauer und/oder der Kalcinationstemperatur eine vorteilhafte Minderung dieses Beitrags bewirkt werden.

Vorzugsweise tragen Poren mit einem Porenradius im Bereich von 0,2 bis 0,4 $\mu$m zum Porengesamtvolumen, bezogen auf das Porengesamtvolumen, mindestens 70 Vol.-%, vorteilhaft mindestens 75 Vol.-%, besonders vorteilhaft mindestens 85 Vol.-% bei.

Alle Angaben in dieser Schrift zu Porengesamtvolumina sowie zu Porendurchmesserverteilungen auf diese Porengesamtvolumina beziehen sich auf Bestimmungen mit der Methode der Quecksilberporosimetrie in Anwendung des Gerätes Auto Pore 9500 der Fa. Micromeritics GmbH, D-41238 Mönchengladbach (Bandbreite 0,003-300 $\mu$m).

Die hohlzylindrischen Katalysatorformkörper weisen bei der Reaktorbefüllung eine hohe Bruchstabilität auf. Die Seitendruckfestigkeit der hohlzylindrischen Katalysatorformkörper beträgt im Allgemeinen 5 bis 15 N, vorzugsweise 6 bis 13 N, besonders bevorzugt 8 bis 11 N. Die experimentelle Bestimmung der Seitendruckfestigkeit wird dabei wie in den Schriften WO 2005/030393 sowie WO 2007/017431 beschrieben durchgeführt.

Die Lagerung der kalcinierten Katalysatorformkörper erfolgt bevorzugt in 120 l Metallfässern, welche im Inneren mit einem Flachsack aus Lupolen und einer Materialdicke von 0,1 mm ausgekleidet sind.

Man bringt den molekularen Sauerstoff und das Alken mit dem Katalysatorfestbett in Kontakt, indem man den molekularen Sauerstoff und das Alken über das Katalysatorfestbett führt. Vorzugsweise führt man ein Reaktionsgas, das den molekularen Sauerstoff und das Alken enthält, über das Katalysatorfestbett und setzt es so zu einem Produktgas um.

Der molekulare Sauerstoff wird dem Verfahren vorzugsweise in Form von Luft zugeführt.

Der Anteil des im Reaktionsgas enthaltenen Alkens wird im Allgemeinen 4 bis 20 Vol.-%, vorzugsweise 5 bis 15 Vol.-%, bevorzugt 5 bis 12 Vol.-%, besonders bevorzugt 5 bis 8 Vol.-% betragen, jeweils bezogen auf das Reaktionsgas.

Vorzugsweise enthält das Reaktionsgas außerdem mindestens ein inertes Verdünnungsgas. Unter inerten Verdünnungsgasen werden solche Gase verstanden, die im Verlauf der Gasphasenoxidation zu mindestens 95 mol-%, vorzugsweise zu mindestens 98 mol-% chemisch unverändert erhalten bleiben. Beispiele für inerte Verdünnungsgase sind $N_2$, $CO_2$, $H_2O$ und Edelgase wie Ar sowie Gemische aus den vorgenannten Gasen. Als inertes Verdünnungsgas wird vorzugsweise molekularer Stickstoff eingesetzt. Das inerte Verdünnungsgas kann z. B. mindestens 20 Vol.-%, vorzugsweise mindestens 40 Vol.-%, bevorzugt mindestens 60 Vol.-%, besonders bevorzugt mindestens 80 Vol.-%, ganz besonders bevorzugt mindestens 95 Vol.-% molekularen Stickstoff enthalten.

Vorzugsweise wird Kreisgas als ein Reaktionsgasbestandteil mitverwendet. Unter Kreisgas wird das Restgas verstanden, das verbleibt, wenn man aus dem Produktgas der Gasphasenoxidation α,β-ungesättigten Aldehyd und/oder α,β-ungesättigte Carbonsäure im Wesentlichen selektiv abtrennt. Dabei ist zu berücksichtigen, dass das erfindungsgemäße Verfahren gegebenenfalls nur die erste Stufe einer zweistufigen Gasphasenoxidation zur α,β-ungesättigten Carbonsäure als der eigentlichen Zielverbindung sein kann, so dass die Kreisgasbildung dann meist erst nach der zweiten Stufe erfolgt. Im Rahmen einer solchen zweistufigen Gasphasenoxidation wird in der Regel das Produktgas der ersten Stufe, gegebenenfalls nach Abkühlung und/oder Sekundärsauerstoffzugabe (in der Regel in Form von Luft), der zweiten Gasphasenoxidation zugeführt.

Das Reaktionsgas kann außerdem mindestens einen weiteren Gasbestandteil enthalten. Der weitere Gasbestandteil ist vorzugsweise ausgewählt unter CO, Methan, Ethan, Propan, Butan, Pentan und $H_2$.

Das Reaktionsgas enthält vorzugsweise Alken : molekularem Sauerstoff : inertem Verdünnungsgas in einem Volumenverhältnis von 1: (1,0 bis 3,0) : (5 bis 25), bevorzugt 1 : (1,5 bis 2,3) : (10 bis 20).

Im Allgemeinen beträgt die Belastung der Schüttung mit im Reaktionsgas enthaltenem Propen mindestens 90 Nl/(lh). Vorzugsweise beträgt die Belastung der Schüttung mit im Reaktionsgas enthaltenem Propen mindestens 100 Nl/(lh), bevorzugt mindestens 130 Nl/(lh) bei einer Temperierzone, bevorzugt mindestens 160 Nl/(lh) bei zwei Temperierzonen. Vorzugsweise beträgt die Belastung der Schüttung mit im Reaktionsgas enthaltenem Propen höchstens 500, bevorzugt höchstens 400, weiterhin bevorzugt höchstens 300, besonders bevorzugt höchstens 250 Nl/(lh). Belastungen im Bereich von 100 bis 300 Nl/(lh), bevorzugt 160 bis 300 Nl/(lh) bei mehrere Temperierzonen, bzw. bevorzugt 100 bis 150 Nl/(lh) bei einer Temperierzone sind besonders zweckmäßig. Unter der Belastung der Schüttung mit im Reaktionsgas enthaltenem Propen wird in dieser Schrift die Menge an Propen in Normlitern (= Nl; das Volumen in Litern, das die entsprechende Propenmenge bei Normalbedingungen, d. h., bei 0 °C und 1 atm (1,01 bar) einnehmen würde) verstanden, die pro Liter Schüttung pro Stunde zugeführt wird (Einheit: Nl/(lh)). Vor- und/oder Nachschüttungen aus Inertmaterial werden bei Belastungsbetrachtungen in dieser Schrift nicht als zur Schüttung gehörig betrachtet; d.h. das Volumen der Schüttung ist das Volumen der die hohlzylindrischen Katalysatorformkörper enthaltenden Schüttung.

Im Allgemeinen herrscht im Reaktionsgas ein Gesamtdruck von 0,5 bis 4 bar, bevorzugt von 1,5 bis 3 bar. Alle Druck-Angaben in dieser Schrift beziehen sich auf absolute Drücke.

Das Verfahren kann z. B. in einem eine Temperierzone aufweisenden Vielkontaktrohr-Festbettreaktor, wie ihn die DE-A 44 31 957, die EP-A 700 714 und die EP-A 700 893 beschreiben, durchgeführt werden. In einem solchen Reaktor liegt ein auf die Kontaktrohre aufgeteiltes Katalysatorfestbett vor. Üblicherweise sind in den vorgenannten Reaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Eine typische Kontaktrohrlänge beläuft sich z. B. auf 3,20 m. Vorzugsweise beläuft sich die im Rohrbündelbehälter untergebrachte Anzahl an Kontaktrohren auf mindestens 1000, vorzugsweise auf mindestens 5000. Häufig beträgt die Anzahl der im Reaktionsbehälter untergebrachten Kontaktrohre 15000 bis 35000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Das Verfahren kann aber auch in einem mehrere Temperierzonen aufweisenden Vielkontaktrohr-Festbettreaktor durchgeführt werden, wie es die DE-A 199 10 506 , die DE-A 103 13 213 , die DE-A 103 13 208 und die EP-A 1 106 598 empfehlen. Eine typische Kontaktrohrlänge im Fall eines zwei Temperierzonen aufweisenden Vielkontaktrohr-Festbettreaktors beträgt 3,50 m. Alles andere gilt im Wesentlichen wie bei dem eine Temperierzone aufweisenden Vielkontaktrohr-Festbettreaktor beschrieben.

Zur Bestimmung des Temperaturprofils entlang des Katalysatorfestbetts in einem Reaktionsrohr eines Rohrbündelreaktors kann dieses eine zentriert durch das Reaktionsrohr von oben nach unten verlaufende Thermohülse aufweisen, in welcher mit Hilfe von in der Thermohülse geführten Thermoelementen die Temperatur über die gesamte Reaktionsrohrlänge ermittelt werden kann. Grundsätzlich könnte jedes in einem Rohrbündelreaktor befindliche und mit dem Katalysatorfestbett beschickte Reaktionsrohr wie vorstehend beschrieben ausgerüstet sein.

Anwendungstechnisch zweckmäßig weist ein Rohrbündelreaktor jedoch nur eine begrenzte Anzahl derartiger Thermoreaktionsrohre oder auch kurz nur "Thermorohre" auf (vgl. z.B. Seite 56 der WO 2007/082827, die EP-A 873783, die EP-A 1270065 und die US 7,534,339 B2).

Da Thermorohre zusätzlich zum Katalysatorfestbett noch die Thermohülse aufnehmen müssen, würden sie bei ansonsten identischer Rohrgestaltung zwar eine gleich große Wärmeaustauschoberfläche, aber einen geringeren freien, vom Katalysatorfestbett einnehmbaren, Querschnitt als ein bloßes "Reaktionsrohr" aufweisen. Dem wird dadurch Rechnung getragen, dass man sie (die Thermorohre) so gestaltet, dass das Verhältnis von freier Querschnittsfläche im Rohr zum Umfang des Rohres bei Thermorohr und Reaktionsrohr gleich sind. Im Übrigen weisen Reaktionsrohr und Thermorohr bei identischer Rohrlänge über ihre Rohrlänge jeweils dieselbe Katalysatorfestbettstruktur auf. Bei der Beschickung mit Katalysatorfestbett ist darüber hinaus darauf zu achten, dass das sich bei der Durchströmung von Reaktionsrohr bzw. Thermorohr mit Reaktionsgasgemisch über die Rohrlänge jeweils einstellende Druckverlustprofil in beiden Rohrtypen einheitlich ist. Über die Geschwindigkeit der Befüllung der Rohre mit den Formkörpern und/oder durch Mitverwendung von zerkleinerten (versplitteten) Formkörpern kann in entsprechender Weise Einfluss genommen werden (vgl. z.B. EP-A 873783 und US 7,534,339 B2). Insgesamt wird auf diese Weise sichergestellt, dass ein Thermorohr und ein Reaktionsrohr entlang der gesamten Rohrlänge zueinander gleiche Verhältnisse von Reaktionswärmeentwicklung im Rohrinneren und Abfuhr von Reaktionswärme aus dem Rohrinneren aufweisen. Das Thermorohr vermag so repräsentativ für viele Reaktionsrohre den Verlauf der Temperatur im Reaktionsrohr abzubilden.

Mit den in den Thermorohren gemessenen Temperaturen lassen sich so die höchste lokale Temperatur des Katalysatorfestbetts und deren Lage im Katalysatorfestbett bestimmen.

Um die Kontaktrohre, auf die das Katalysatorfestbett aufgeteilt ist, wird in jeder Temperierzone ein Wärmeaustauschmittel geführt. Bevorzugte Wärmeaustauschmittel sind Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Die Eingangstemperatur des Wärmeaustauschmittels wird vorzugsweise auf 280°C bis 420°C, bevorzugt auf 300°C bis 400°C , besonders bevorzugt auf 320°C bis 380°C eingestellt.

Das Wärmeaustauschmittel kann über die jeweilige Temperierzone betrachtet, im Gleichstrom oder im Gegenstrom zum Reaktionsgasgemisch geführt werden. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperierzone bis zur Austrittstelle aus der Temperierzone um 0 bis 15 °C, häufig 1 bis 10 °C, oder 2 bis 8 °C, oder 3 bis 6 °C ansteigt. Innerhalb der Temperierzone wird das Wärmeaustauschmittel bevorzugt mäanderförmig geführt.

Die Inbetriebnahme des Verfahrens kann z. B. wie in der DE-A 103 37 788 oder wie in der DE-A 102009047291 beschrieben erfolgen.

Ein durch das erfindungsgemäße Verfahren hergestellte $\alpha,\beta$-ungesättigte Aldehyd kann in einer zweiten Stufe weiter zur $\alpha,\beta$-ungesättigten Carbonsäure umgesetzt werden.

Eine mit der Gasphasenoxidation von Alken (Propen, Isobuten) zu $\alpha,\beta$-ungesättigtem Aldehyd (Acrolein, Methacrolein) einhergehende Nebenproduktbildung an $\alpha,\beta$-ungesättigter Carbonsäure (Acrylsäure, Methacrylsäure) ist in der Regel nicht unerwünscht. Die Wertprodukte (Aldehyd und Carbonsäure) lassen sich in einem späteren Verfahrensschritt trennen.

Beispiele

Herstellung von hohlzylindrischen Katalysatorformkörpern

Lösung A: In einem zur Atmosphäre (1 atm, 1,01 bar) offenen, temperierbaren und mit einem Ankerrührer ausgestatteten Behälter aus Edelstahl (Innenvolumen = 5 dm$^3$) wurden 1060,5 g einer wässrigen Kobalt-(II)-nitratlösung (12,3 Gew.-% Co, 27 Gew.-% Nitrat (NO$^{3-}$)), pH = 1, hergestellt durch Lösen von Kobaltmetall der Fa. MFT Metals & Ferro-Allogs Trading GmbH, D-41474 Viersen, Reinheit > 99,6 Gew.-% Co, < 0,3 Gew.-% Ni, < 100 mg/kg Fe, < 50 mg/kg Cu in Salpetersäure) vorgelegt und unter Rühren (150 U/min) auf 60 °C erwärmt. Unter fortgesetztem Rühren (150 U/min) und fortgesetzter Temperierung auf 60 °C wurden 225,0 g an kristallinem Eisen-(III)-nitratnonahydrat (13,9 Gew.-% Fe, < 0,4 Gew.-% Alkalimetalle, < 0,01 Gew.-% Chlorid, < 0,02 Gew.-% Sulfat, der Fa. Dr. Paul Lohmann GmbH, D-81857 Emmerthal) zudosiert und 10 min bei 60 °C nachgerührt. Zur dabei resultierenden wässrigen Lösung wurden 278,6 g einer 60 °C warmen, wässrigen, salpetersauren Bismutnitratlösung (12,0 Gew.-% Bi, 13 Gew.-% Nitrat, hergestellt durch Lösen von Bismutmetall der Fa. Sidech S. A., BE-1495 Tilly, Reinheit > 99,997 Gew.-% Bi, < 7 mg/kg Pb, je < 5 mg/kg Ni, Ag, Fe, je < 3 mg/kg Cu, Sb und < 1 mg/kg Cd, Zn in Salpetersäure) gegeben und 10 min bei 60 °C nachgerührt.

Lösung B: In einem zur Atmosphäre (1 atm, 1,01 bar) offenen, temperierbaren und mit einem Ankerrührer ausgestatteten Behälter aus Edelstahl (Innenvolumen = 10 dm$^3$) wurden 4750 g vollentsalztes Wasser vorgelegt und unter Rühren (150 U/min) auf 60 °C erhitzt. Anschließend wurden 2,53 g einer 33 gew.-%igen wässrigen Lösung von KOH in Wasser, die eine Temperatur von 60 °C aufwies, zugegeben. Nach 1-minütigem Nachrühren bei 60 °C wurde unter Beibehalt der 60 °C 568,53 g Ammoniumheptamolybdattetrahydrat (weiße Kristalle mit einer Körnung d < 1 mm, 54 Gew.-% Mo, 7,0-8,5 Gew.-% NH$_3$, max. 150 mg/kg Alkalimetalle, der Fa. H. C. Starck, D-38642 Goslar) portionsweise eingerührt und die dabei resultierende wässrige Lösung bei 60 °C 20 min nachgerührt (150 U/min).

Die 60 °C warme Lösung A wurde mit Hilfe einer Schlauchpumpe (Typ: BVP, Firma: Ismatec SA, Labortechnik-Analytik, Feldeggstrasse 6, CH-8152 Glattbrugg, Einstellung: 320 Skalenteile) innerhalb von 15 min kontinuierlich in die nun mit einem Ultra-Turrax (Firma Janke & Kunkel GmbH & Co. KG-IKA-Labortechnik, Janke & Kunkel-Str. 10, DE-79219 Staufen, Schaft-Typ: 550KR-G45 fein, Schaftrohrdurchmesser: 25 mm, Statordurchmesser: 45 mm, Rotordurchmesser: 40 mm, Einstellung: Stufe 5) intensiv gerührte 60 °C warme Lösung B eindosiert. Die Aufgabe der Lösung A erfolgte dabei auf der Höhe des Rotors des Ultra-Turrax Rührers um etwa 0,5 bis 1 cm von der Außenkante des Rotors des Utra-Turrax Rührers versetzt. Die entstehende wässrige Suspension wurde noch 15 min bei 60 °C nachgerührt.

Die wässrige Suspension wurde mit 48,5 g eines auf 60 °C erwärmten Kieselgels der Fa. Grace vom Typ Ludox TM50 (24,4 Gew.-% Si, Dichte: 1,29 g/cm$^3$, pH: 8,5 bis 9,5, Alkaligehalt max. 0,5 Gew.-%) versetzt, weitere 15 min bei 60 °C gerührt und anschließend in einem Sprühturm vom Typ Mobile Minor™ 2000 (MM-I) der Firma Niro A/S, Gladsaxevej 305, 2860 Soborg, Dänemark, mit einem Zentrifugalzerstäuber vom Typ F01A und einem Zerstäuberrad vom Typ SL24-50 im Heißluftgleichstrom innerhalb von 90 bis 140 min sprühgetrocknet (Gaseintrittstemperatur: 350 +- 10 °C, Gasaustrittstemperatur: 140 +- 5 °C). Der noch nicht sprühgetrocknete Anteil wurde stets bei 60 °C weitergerührt. Die Einstellung der Drehzahl des Zerstäuberrads betrug 25000 U/min. Es wurden so etwa 942 g an orange-braunem Sprühpulver erhalten. Die Restfeuchte des Sprühpulvers (Restfeuchtebestimmung mittels Mikrowellen) lagen bei 3 Gew.-% bezogen auf das Gesamtgewicht des Sprühpulvers.

In das Sprühpulver wurden in einem Rhönradmischer (Raddurchmesser: 650 mm, Trommelvolumen: 5 l), bezogen auf das Gewicht des Sprühpulvers, 1 Gew.-% feinteiliger Graphit der Sorte TIMREX T44 der Fa. Timcal Ltd., CH-6743 Bodio (vgl. WO 2008/087116) homogen verteilt eingemischt (Drehzahl: 30 U/min, Einmischdauer: 30 min). In einem Laborkalander mit 2 gegenläufigen Stahlwalzen (Walzendurchmesser: 10 cm; zur Zwischenkompaktierung genutzte Walzenlänge: 13,5 cm; Walzendrehzahl: 10 U/min) wurde das homogene Gemisch bei einem Pressdruck von 9 bar kompaktiert und anschließend durch ein Sieb mit quadratischen Maschen (Kantenlänge = 0,8 mm) gedrückt. In das so vergröberte Sprühpulver wurden im vorstehend beschriebenen Rhönradmischer (30 U/min, 30 min Einmischdauer), bezogen auf das Gewicht des vergröberten Sprühpulvers, weitere 2,5 Gew.-% desselben feinteiligen Graphits eingemischt. Anschließend wurde das so erhaltene feinteilige innige Trockengemisch wie in der DE-A 10200804093 beschrieben, mit einem Kilian Rundläufer (9-fach-Tablettiermaschine) vom Typ 5100 (Fa. Kilian, D-50735 Köln) unter Stickstoffatmosphäre und einer Umgebungstemperatur von 25 °C zu hohlzylindrischen Formkörpern der Geometrie AD x H x ID = 5 mm x 3 mm x 2 mm, verdichtet (tablettiert). Die Einstellungen der Tablettiermaschine erfolgten dabei derartig, dass das Tablettengewicht 119 mg betrug, was einer Tablettendichte von 2,4 kg/l entspricht.

Formkörper der Geometrie AD x H x ID = 5 mm x 3 mm x 2,5 mm, AD x H x ID = 5 mm x 3 mm x 3 mm, AD x H x ID = 6 mm x 3 mm x 2 mm, AD x H x ID = 6 mm x 3 mm x 3 mm und AD x H x ID = 4 mm x 3 mm x 2 mm wurden entsprechend hergestellt. Die Steuergröße bei der Einstellung der Tablettiermaschine war dabei jeweils eine Zieltablettendichte von 2,4 kg/l.

Die thermische Vorbehandlung und Kalcination der so erhaltenen Formkörper erfolgte wie auf Seite 29 der DE 10 2011 084 040 A1 für ringförmige Vollkatalysatorvorläuferformkörper beschrieben.

**Gasphasenoxidation von Propen zu Acrolein und Acrylsäure unter Verwendung eines Katalysatorfestbetts mit aufeinanderfolgenden Reaktionszonen**

Ein Reaktionsrohr (Edelstahl Typ 1.4541 (EU Normnummer EN 10088-3; 33,7 mm Außendurchmesser; 2 mm Wandstärke; 29,7 mm Innendurchmesser; 400 cm Länge, 4 mm Thermohülse) wurde von unten nach oben wie folgt beschickt:

| | |
|---|---|
| Abschnitt 1: | 90 cm Länge |
| | Vorschüttung aus Steatit-Ringen der Geometrie 7 mm x 3 mm x 4 mm (Außendurchmesser x Länge x Innendurchmesser; Steatit C220 der Fa. CeramTec); |
| Reaktionszone: | bestehend aus 2-3 Abschnitten (siehe Tabelle 1; dabei liegt Zone 1 dem Reaktoreingang am nächsten) |
| Abschnitt 2: | 57 cm Länge Nachschüttung aus denselben Steatit-Ringen wie in Abschnitt 1; |

Abschnitt 3:        46 cm Länge Leerrohr

In Tabelle 1 ist die Struktur der Reaktionszonen dargestellt. Zur Einstellung der relativen volumenspezifischen Katalysatoraktivität wurden Katalysatorformkörper mit inerten Steatitringen der Geometrie 5 mm x 3 mm x 2 mm (Außendurchmesser x Länge x Innendurchmesser, C220 Steatit von CeramTec) verdünnt.
Durch das jeweilige wie vorstehend beschrieben beschickte Reaktionsrohr wurde von oben nach unten durch das Reaktionsrohr strömend ein Reaktionsgasgemisch geführt, das folgende Gehalte aufwies:

- 5,2 bis 6,5 Vol.-% Propen,
- 3 bis 3,5 Vol.-% $H_2O$,
- 0,3 bis 0,6 Vol.-% CO,
- 0,6 bis 1,0 Vol.-% $CO_2$,
- 0,02 bis 0,05 Vol.-% Acrolein,
- 9,8 bis 10,4 Vol.-% $O_2$ und

als Restmenge ad 100 % molekularer Stickstoff.
Die Belastung des Katalysatorfestbetts mit Propen betrug jeweils 100 Nl/(lh).
Das Reaktionsrohr war über seine Länge jeweils von einem gerührten und von außen elektrisch beheizten Salzbad (Gemisch aus 53 Gew.-% Kaliumnitrat, 40 Gew.-% Natriumnitrit und 7 Gew.-% Natriumnitrat; 50 kg Salzschmelze) umspült (die Strömungsgeschwindigkeit am Rohr war 3 $m^3$/h (in der Ebene senkrecht zur Längsachse des Rohres)).
Die Salzbadtemperatur $T^B$ (°C) (mit der das Salzbad zugeführt wurde) wurde in allen Fällen so eingestellt, dass ein auf den einfachen Durchgang des Reaktionsgasgemischs durch das Katalysatorfestbett bezogener Propenumsatz von 96,0 mol-% resultierte. Entlang des Reaktionsrohres änderte sich die Salzbadtemperatur infolge Zuheizung nicht (es wurde vom Salzbad mehr Wärme abgestrahlt wie vom Reaktionsrohr an das Salzbad abgegeben). Die Zuführtemperatur des Reaktionsgasgemischs (am Eingang in das Reaktionsrohr) war jeweils auf die jeweilige Salzbadtemperatur eingestellt.
Die Temperatur im Katalysatorbett wurde durch ein Thermoelement, welches in einer Thermohülse, die sich im Inneren des Reaktorrohres befand, platziert war und mit Hilfe einer Zugmaschine von unten nach oben im Reaktorbett geschoben wurde, kontinuierlich gemessen. Die maximale Temperatur dieser Messung entsprach der Heißpunkttemperatur $T^H$.

Tabelle 1:

| Bsp. | Reaktionszone: Länge [m] | | | Katalysatorform körper: Abmessungen [mm] $(AD^{1)}xH^{2)}xID^{3)})$ | | | Verdünnung mit Inertmaterial [Gew.-%] | | | Relative volumenspezifische Katalysatoraktivität [%] | | |
|---|---|---|---|---|---|---|---|---|---|---|---|---|
| | Zone 1 | Zone 2 | Zone 3 | Zone 1 | Zone 2 | Zone 3 | Zone 1 | Zone 2 | Zone 3 | Zone 1 | Zone 2 | Zone 3 |
| 1 | 0,1 | 0,9 | 1,8 | 5x3x2 | 5x3x3 | 5x3x2 | 20 | 20 | 0 | 80 | 56 | 100 |
| 2 | 0,1 | 0,9 | 1,8 | 5x3x2 | 5x3x3 | 5x2x2 | 20 | 20 | 0 | 80 | 56 | 100 |
| 3*) | 0,1 | 0,9 | 1,8 | 5x3x2 | 5x3x2 | 5x3x2 | 20 | 40 | 0 | 80 | 60 | 100 |
| 4*) | 1 | 1,8 | | 5x3x2 | 5x3x2 | - | 40 | 0 | - | 60 | 100 | |
| 5 | 0,1 | 0,9 | 1,8 | 5x3x2 | 4x3x2 | 5x3x2 | 20 | 40 | 0 | 80 | 60 | 100 |

*): Vergleichsbeispiel
1): Außendurchmesser
2): Höhe
3): Innendurchmesser

Tabelle 2:

| Bsp. | $T^{B \, 1)}$ | $T^{H \, 2)}$ | $y^{H \, 3)}$ | $S^{\, 4)}$ |
|------|------|------|------|------|
|  | (°C) | (°C) | (m) | (mol%) |
| 2 | 346 | 405 | 0,4 | 96,65 |
| 3*) | 346 | 396 | 0,35 | 96,3 |
| 1) Salzbadtemperatur<br>2) Heißpunkttemperatur<br>3) Heißpunktposition (der Nullpunkt ist der Beginn der Reaktionszone); Die Position 0,4 bzw. 0,35 zeigt, dass sich der Heißpunkt in beiden Fällen in der 2. Zone befindet.<br>4) Selektivität der Wertproduktbildung (Wertprodukte: Acrolein und Acrylsäure) bezogen auf das umgesetzte Propen | | | | |

Unter der Selektivität der Wertproduktbildung (S (mol%)) wird in dieser Schrift verstanden:

$$S = \frac{\text{Molzahl Propen umgesetzt zu Acrolein und Acrylsäure x 100}}{\text{Molzahl Propen insgesamt umgesetzt}}$$

(die Umsatzzahlen jeweils bezogen auf einen einmaligen Durchgang des Reaktionsgasgemischs durch das Katalysatorfestbett).

**Gasphasenoxidation von Propen zu Acrolein und Acrylsäure in einem Katalysatorfestbett mit zwei aufeinanderfolgenden Temperierzonen**

Ein Thermoreaktionsrohr (V2A Stahl; 33,7 mm Außendurchmesser, 2 mm Wandstärke, 29,7 mm Innendurchmesser, Länge: 350 cm, sowie ein in der Thermorohrmitte zentriert von oben nach unten verlaufendes Thermoschutzrohr (Thermohülse) zur Aufnahme eines Thermoelements, mit dem auf der gesamten Rohrlänge die Reaktionstemperatur (die effektive Katalysatorfestbetttemperatur) ermittelt werden konnte) wurde von oben nach unten wie folgt beschickt:

Abschnitt 1:                                           50 cm Länge
• Steatitringe der Geometrie 7 mm x 7 mm x 4 mm (Außendurchmesser x Länge x Innendurchmesser, Steatit C220 der Fa. CeramTec) als Vorschüttung.
Abschnitt 2:                                           10 cm Länge
• Katalysatorbeschickung mit einem homogenen Gemisch bestehend aus 20 Gew.-% Steatitringen der Geometrie 5 mm × 3 mm × 2 mm (Außendurchmesser x Länge x Innendurchmesser; Steatit C220 der Fa. CeramTec), 80 Gew.-% Katalysatorformkörpern der Geometrie AD x H x ID = 5 mm x 3 mm x 2 mm.
Abschnitt 3:                                           130 cm Länge
• Katalysatorbeschickung mit einem homogenen Gemisch bestehend aus 20 Gew.-% Steatitringen der Geometrie 5 mm × 3 mm × 2 mm (Außendurchmesser x Länge x Innendurchmesser; Steatit C220 der Fa. CeramTec), 80 Gew.-% Katalysatorformkörpern der Geometrie AD x H x ID = 5 mm x 3 mm x 3 mm.
Section 4:                                             length 160 cm
• Katalysatorbeschickung mit 100 Gew.-% Katalysatorformkörpern der Geometrie AD x H x ID = 5 mm x 3 mm x 2 mm.

Das Thermoreaktionsrohr soll das Verhalten eines bloßen Reaktionsrohres aus entsprechendem Material und mit identischer Rohrlänge abbilden, dessen Innendurchmesser bei 2 mm Wandstärke 26 mm beträgt und das in entsprechender Weise wie das Thermoreaktionsrohr beschickt wird.
Von oben nach unten wurden die ersten 175 cm des Thermoreaktionsrohrs mittels eines über die 175 cm zum Reaktionsgasgemisch im Gegenstrom gepumpten Salzbades A thermostatisiert, das mit der Temperatur T_A zugeführt wurde. Die zweiten 175 cm wurden mittels eines in entsprechender Weise im Gegenstrom gepumpten Salzbades B thermostatisiert, das mit der Temperatur T_B zugeführt wurde. Über die jeweilige Temperaturzone war die jeweilige Salzbadtemperatur im Wesentlichen konstant.
Die beiden Salzbäder A, B bestanden jeweils aus 53 Gew.-% Kaliumnitrat, 40 Gew.-% Natriumnitrit und 7 Gew.-% Natriumnitrat. In den beiden 175 cm langen Rohrabschnitten erfolgte die Anströmung des Reaktionsrohres im Wesentlichen vertikal zur Strömungsrichtung des Reaktionsgases. Die Strömungsgeschwindigkeit beider Salzschmelzen war so bemessen, dass eine weitere Erhöhung im Wesentlichen keine Verbesserung des Wärmedurchgangs aus dem Thermorohrinneren ins Salzbad bewirkt hätte (vgl. EP-A1547994).

Das wie vorstehend beschrieben beschickte Thermorohr wurde mit einem Reaktionsgaseingangsgemisch beschickt, das nachfolgende Gehalte aufwies und aus Luft (als Sauerstoffquelle), chemical grade Propen und Kreisgas erzeugt wurde:

- 6 bis 6,5 Vol.-% Propen
- 0,7 bis 1,2 Vol.-% $H_2O$,
- 0,4 bis 0,6 Vol.% CO,
- 0,7 bis 1,1 Vol.-% $CO_2$,
- 0,01 bis 0,04 Vol.-% Acrolein,
- 0,005 bis 0,015 Vol.-% Ethen,
- 0,025 bis 0,035 Vol.-% Propan,
- 10,8 bis 11,7 Vol.-% $O_2$ und
- wenigstens 77 Vol.-% $N_2$.

Die Belastung des Katalysatorfestbetts mit im Reaktionsgaseingangsgemisch enthaltenem Propen lag stets im Bereich von 150 bis 200 NI/(Ih). Der Druck am Eingang des Thermoreaktionsrohres betrug dabei im Wesentlichen 1.5 bis 2.5 barG. Die Temperatur T_A betrug 325 °C, die Temperatur T_B betrug 341 °C.

**Patentansprüche**

1.  Verfahren zur Herstellung eines $\alpha,\beta$-ungesättigten Aldehyds und/oder einer $\alpha,\beta$-ungesättigten Carbonsäure durch Gasphasenoxidation eines Alkens mit molekularem Sauerstoff an einem Katalysatorfestbett, das eine Schüttung hohlzylindrischer Katalysatorformkörper umfasst, deren aktive Masse ein Multimetalloxid ist, **dadurch gekennzeichnet, dass**

    (i) das Katalysatorfestbett mindestens drei aufeinanderfolgende Reaktionszonen umfasst;
    (ii) die höchste lokale Temperatur des Katalysatorfestbetts nicht in der dem Reaktorausgang nächsten Reaktionszone auftritt;
    (iii) die höchste lokale Temperatur des Katalysatorfestbetts nicht in der dem Reaktoreingang nächsten Reaktionszone auftritt; und
    (iv) der Wert WS gemäß der nachstehenden Gleichung in derjenigen Reaktionszone, in der die höchste lokale Temperatur des Katalysatorfestbetts auftritt, kleiner ist als in den anderen Reaktionszonen,

    $$WS = \frac{AD-ID}{2}$$

    worin AD für den Außendurchmesser und ID für den Innendurchmesser des Katalysatorformkörpers steht.

2.  Verfahren nach Anspruch 1, **dadurch gekennzeichnet, dass** das Multimetalloxid mindestens die Elemente Eisen, Bismut und mindestens eines der Elemente Molybdän und Wolfram umfasst.

3.  Verfahren nach Anspruch 2, **dadurch gekennzeichnet, dass** das Multimetalloxid der Formel (I) entspricht,

    $$Mo_{12}Bi_aFe_bX^1_cX^2_dX^3_eX^4_fO_n \qquad (I),$$

    worin

    $X^1$ für Nickel und/oder Kobalt steht,
    $X^2$ für Thallium, ein Alkalimetall und/oder ein Erdalkalimetall steht,
    $X^3$ für Zink, Phosphor, Arsen, Bor, Antimon, Zinn, Cer, Blei, Vanadium, Chrom und/oder Wolfram steht,
    $X^4$ für Silicium, Aluminium, Titan und/oder Zirkonium steht,
    a für eine Zahl im Bereich von 0,2 bis 5 steht,
    b für eine Zahl im Bereich von 0,01 bis 10 steht,

c für eine Zahl im Bereich von 0 bis 10 steht,
d für eine Zahl im Bereich von 0 bis 2 steht,
e für eine Zahl im Bereich von 0 bis 8 steht,
f für eine Zahl im Bereich von 0 bis 10 steht, und
n für eine Zahl steht, die durch die Wertigkeit und Häufigkeit der von Sauerstoff verschiedenen Elemente in (I) bestimmt wird;

oder der Formel (II) entspricht,

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (II),$$

worin

$Y^1$ für Bismut oder für Bismut und mindestens eines der Elemente Tellur, Antimon, Zinn und Kupfer steht,
$Y^2$ für Molybdän oder Wolfram, oder für Molybdän und Wolfram steht,
$Y^3$ für ein Alkalimetall, Thallium und/oder Samarium steht,
$Y^4$ für ein Erdalkalimetall, Nickel, Kobalt, Mangan, Zink, Zinn, Cadmium und/oder Quecksilber steht,
$Y^5$ für Eisen oder für Eisen und mindestens eines der Elemente Vanadium, Chrom und Cer steht,
$Y^6$ für Phosphor, Arsen, Bor, Antimon und/oder Bismut steht,
$Y^7$ für ein seltenes Erdmetall, Titan, Zirkonium, Niob, Tantal, Rhenium, Ruthenium, Rhodium, Kupfer, Silber, Gold, Aluminium, Gallium, Indium, Silicium, Germanium, Blei, Thorium und/oder Uran steht,
$Y^8$ für Molybdän oder Wolfram, oder für Molybdän und Wolfram steht,
a' für eine Zahl im Bereich von 0,01 bis 8 steht,
b' für eine Zahl im Bereich von 0,1 bis 30 steht,
c' für eine Zahl im Bereich von 0 bis 4 steht,
d' für eine Zahl im Bereich von 0 bis 20 steht,
e' für eine Zahl größer 0 im Bereich von 0 bis 20 steht,
f' für eine Zahl im Bereich von 0 bis 6 steht,
g' für eine Zahl im Bereich von 0 bis 15 steht,
h' für eine Zahl im Bereich von 8 bis 16 steht,
x' und y' für Zahlen stehen, die durch die Wertigkeit und Häufigkeit der von Sauerstoff verschiedenen Elemente in (II) bestimmt werden, und
p und q für Zahlen stehen, deren Verhältnis p/q 0,1 bis 10 beträgt.

4. Verfahren nach einem der vorhergehenden Ansprüche, **dadurch gekennzeichnet, dass** das geometrische Volumen der Katalysatorformkörper kleiner als 80 mm$^3$ ist.

5. Verfahren nach einem der vorhergehenden Ansprüche, **dadurch gekennzeichnet, dass** die volumenspezifische Katalysatoraktivität derjenigen Reaktionszone, in der die höchste lokale Temperatur des Katalysatorfestbetts auftritt, niedriger ist als in den anderen Reaktionszonen.

6. Verfahren nach einem der vorhergehenden Ansprüche, **dadurch gekennzeichnet, dass** die Katalysatoraktivmassendichte derjenigen Reaktionszone, in der die höchste lokale Temperatur des Katalysatorfestbetts auftritt, niedriger ist als in den anderen Reaktionszonen.

7. Verfahren nach einem der vorhergehenden Ansprüche, **dadurch gekennzeichnet,**
**dass** der Außendurchmesser der Katalysatorformkörper in derjenigen Reaktionszone, in der die höchste lokale Temperatur des Katalysatorfestbetts auftritt, kleiner ist als der Außendurchmesser der Katalysatorformkörper in den anderen Reaktionszonen und der Innendurchmesser der Katalysatorformkörper in allen Reaktionszonen gleich ist; oder
**dass** der Innendurchmesser der Katalysatorformkörper in derjenigen Reaktionszone, in der die höchste lokale Temperatur des Katalysatorfestbetts auftritt, größer ist als der Innendurchmesser der Katalysatorformkörper in den anderen Reaktionszonen und der Außendurchmesser der Katalysatorformkörper in allen Reaktionszonen gleich ist.

8. Verfahren nach einem der vorhergehenden Ansprüche, **dadurch gekennzeichnet, dass** das Katalysatorfestbett drei aufeinanderfolgende Reaktionszonen umfasst, wobei

(i) die erste Reaktionszone 2 bis 5 % des Volumens des Katalysatorfestbetts,

(ii) die zweite Reaktionszone 25 bis 45 % des Volumens des Katalysatorfestbetts, und
(iii) die dritte Reaktionszone 50 bis 73 % des Volumens des Katalysatorfestbetts ausmacht.

9. Verfahren nach einem der vorhergehenden Ansprüche, **dadurch gekennzeichnet, dass** das Katalysatorfestbett drei aufeinanderfolgende Reaktionszonen umfasst, wobei

(i) die erste Reaktionszone 70 bis 90 % der volumenspezifischen Katalysatoraktivität der dritten Reaktionszone aufweist, und
(ii) die zweite Reaktionszone 50 bis 70 % der volumenspezifischen Katalysatoraktivität der dritten Reaktionszone aufweist.

10. Verfahren nach einem der vorhergehenden Ansprüche, **dadurch gekennzeichnet, dass** das Katalysatorfestbett drei aufeinanderfolgende Reaktionszonen umfasst, wobei

(i) die erste Reaktionszone 70 bis 90 % der Katalysatoraktivmassendichte der dritten Reaktionszone aufweist, und
(ii) die zweite Reaktionszone 50 bis 70 % der Katalysatoraktivmassendichte der dritten Reaktionszone aufweist.

11. Verfahren nach einem der vorhergehenden Ansprüche, **dadurch gekennzeichnet, dass** die hohlzylindrischen Katalysatorformkörper einen Außendurchmesser von 3 bis 5 mm, eine Höhe von 2 bis 4 mm und einen Innendurchmesser von 1 bis 4 mm aufweisen.

12. Verfahren nach einem der vorhergehenden Ansprüche, **dadurch gekennzeichnet, dass** das Katalysatorfestbett drei aufeinanderfolgende Reaktionszonen umfasst, wobei

(i) die erste Reaktionszone Katalysatorformkörper mit einem Außendurchmesser von 5 mm und einem Innendurchmesser von 2 mm,
(ii) die zweite Reaktionszone Katalysatorformkörper mit einem Außendurchmesser von 5 mm und einem Innendurchmesser von 3 mm, und
(iii) die dritte Reaktionszone Katalysatorformkörper mit einem Außendurchmesser von 5 mm und einem Innendurchmesser von 2 mm umfasst,

wobei die Höhe der Katalysatorformkörper je 2,5 mm oder 3 mm beträgt.

13. Verfahren nach einem der vorhergehenden Ansprüche, **dadurch gekennzeichnet, dass** das Katalysatorfestbett drei aufeinanderfolgende Reaktionszonen umfasst, wobei

(i) die erste Reaktionszone Katalysatorformkörper mit einem Außendurchmesser von 5 mm und einem Innendurchmesser von 2 mm,
(ii) die zweite Reaktionszone Katalysatorformkörper mit einem Außendurchmesser von 4 mm und einem Innendurchmesser von 2 mm, und
(iii) die dritte Reaktionszone Katalysatorformkörper mit einem Außendurchmesser von 5 mm und einem Innendurchmesser von 2 mm umfasst,

wobei die Höhe der Katalysatorformkörper je 2,5 mm oder 3 mm beträgt.

14. Verfahren nach einem der vorhergehenden Ansprüche, **dadurch gekennzeichnet, dass** eine oder mehrere Reaktionszonen des Katalysatorfestbetts Mischungen aus Katalysatorformkörpern und Inertmaterial enthält, wobei Steatit als Inertmaterial eingesetzt wird und das Inertmaterial in Form hohlzylindrischer Formkörper vorliegt, deren Abmessungen im Wesentlichen dem Außendurchmesser, der Höhe und dem Innendurchmesser der in der jeweiligen Reaktionszone eingesetzten Katalysatorformkörper entsprechen.

15. Verfahren nach einem der vorhergehenden Ansprüche zur Herstellung von Acrolein durch Gasphasenoxidation von Propen.

**Claims**

1.  A process for preparing an α,β-unsaturated aldehyde and/or an α,β-unsaturated carboxylic acid by gas phase oxidation of an alkene with molecular oxygen over a fixed catalyst bed comprising a bed of hollow cylindrical shaped catalyst bodies having a multimetal oxide active composition, wherein

    (i) the fixed catalyst bed comprises at least three successive reaction zones;
    (ii) the highest local temperature in the fixed catalyst bed does not occur in the reaction zone closest to the reactor outlet;
    (iii)the highest local temperature in the fixed catalyst bed does not occur in the reaction zone closest to the reactor inlet; and
    (iv) the value WT according to the following equation in that reaction zone in which the highest local temperature in the fixed catalyst bed occurs is lower than in the other reaction zones:

    $$WT = \frac{ED - ID}{2}$$

    in which ED is the external diameter and ID is the internal diameter of the shaped catalyst body.

2.  The process according to claim 1, wherein the multimetal oxide comprises at least the elements iron, bismuth and at least one of the elements molybdenum and tungsten.

3.  The process according to claim 2, wherein the multimetal oxide corresponds to the formula (I)

    $$Mo_{12}Bi_aFe_bX^1_cX^2_dX^3_eX^4_fO_n \qquad (I)$$

    in which

    $X^1$ is nickel and/or cobalt,
    $X^2$ is thallium, an alkali metal and/or an alkaline earth metal,
    $X^3$ is zinc, phosphorus, arsenic, boron, antimony, tin, cerium, lead, vanadium, chromium and/or tungsten,
    $X^4$ is silicon, aluminum, titanium and/or zirconium,
    a is a number in the range from 0.2 to 5,
    b is a number in the range from 0.01 to 10,
    c is a number in the range from 0 to 10,
    d is a number in the range from 0 to 2,
    e is a number in the range from 0 to 8,
    f is a number in the range from 0 to 10, and
    n is a number which is determined by the valency and frequency of the elements other than oxygen in (I);

    or corresponds to the formula (II)

    $$[Y^1_{a'}Y^2_{b'}O_{x'}]_p[Y^3_{c'}Y^4_{d'}Y^5_{e'}Y^6_fY^7_gY^8_{h'}O_{y'}]_q \qquad (II)$$

    in which

    $Y^1$ is bismuth or is bismuth and at least one of the elements tellurium, antimony, tin and copper,
    $Y^2$ is molybdenum or tungsten, or is molybdenum and tungsten,
    $Y^3$ is an alkali metal, thallium and/or samarium,
    $Y^4$ is an alkaline earth metal, nickel, cobalt, manganese, zinc, tin, cadmium and/or mercury,
    $Y^5$ is iron or is iron and at least one of the elements vanadium, chromium and cerium,
    $Y^6$ is phosphorus, arsenic, boron, antimony and/or bismuth,
    $Y^7$ is a rare earth metal, titanium, zirconium, niobium, tantalum, rhenium, ruthenium, rhodium, copper, silver, gold, aluminum, gallium, indium, silicon, germanium, lead, thorium and/or uranium,
    $Y^8$ is molybdenum or tungsten, or is molybdenum and tungsten,
    a' is a number in the range from 0.01 to 8,
    b' is a number in the range from 0.1 to 30,

c' is a number in the range from 0 to 4,
d' is a number in the range from 0 to 20,
e' is a number greater than 0 in the range from 0 to 20,
f' is a number in the range from 0 to 6,
g' is a number in the range from 0 to 15,
h' is a number in the range from 8 to 16,
x' and y' are numbers which are determined by the valency and frequency of the elements other than oxygen in (II), and
p and q are numbers whose ratio p/q is 0.1 to 10.

4. The process according to any of the preceding claims, wherein the geometric volume of the shaped catalyst bodies is less than 80 mm$^3$.

5. The process according to any of the preceding claims, wherein the volume-specific catalyst activity of that reaction zone in which the highest local temperature in the fixed catalyst bed occurs is lower than in the other reaction zones.

6. The process according to any of the preceding claims, wherein the catalyst active composition density in that reaction zone in which the highest local temperature in the fixed catalyst bed occurs is lower than in the other reaction zones.

7. The process according to any of the preceding claims, wherein
the external diameter of the shaped catalyst bodies in that reaction zone in which the highest local temperature in the fixed catalyst bed occurs is less than the external diameter of the shaped catalyst bodies in the other reaction zones, and the internal diameter of the shaped catalyst bodies in all the reaction zones is the same; or the internal diameter of the shaped catalyst bodies in that reaction zone in which the highest local temperature in the fixed catalyst bed occurs is greater than the internal diameter of the shaped catalyst bodies in the other reaction zones, and the external diameter of the shaped catalyst bodies in all the reaction zones is the same.

8. The process according to any of the preceding claims, wherein the fixed catalyst bed comprises three successive reaction zones, and

(i) the first reaction zone makes up 2 to 5% of the volume of the fixed catalyst bed,
(ii) the second reaction zone makes up 25 to 45% of the volume of the fixed catalyst bed, and
(iii) the third reaction zone makes up 50 to 73% of the volume of the fixed catalyst bed.

9. The process according to any of the preceding claims, wherein the fixed catalyst bed comprises three successive reaction zones, and

(i) the first reaction zone has 70 to 90% of the volume-specific catalyst activity of the third reaction zone, and
(ii) the second reaction zone has 50 to 70% of the volume-specific catalyst activity of the third reaction zone.

10. The process according to any of the preceding claims, wherein the fixed catalyst bed comprises three successive reaction zones, and

(i) the first reaction zone has 70 to 90% of the catalyst active composition density of the third reaction zone, and
(ii) the second reaction zone has 50 to 70% of the catalyst active composition density of the third reaction zone.

11. The process according to any of the preceding claims, wherein the hollow cylindrical shaped catalyst bodies have an external diameter of 3 to 5 mm, a height of 2 to 4 mm and an internal diameter of 1 to 4 mm.

12. The process according to any of the preceding claims, wherein the fixed catalyst bed comprises three successive reaction zones, and

(i) the first reaction zone comprises shaped catalyst bodies having an external diameter of 5 mm and an internal diameter of 2 mm,
(ii) the second reaction zone comprises shaped catalyst bodies having an external diameter of 5 mm and an internal diameter of 3 mm, and
(iii) the third reaction zone comprises shaped catalyst bodies having an external diameter of 5 mm and an internal diameter of 2 mm, where the height of the shaped catalyst bodies in each case is 2.5 mm or 3 mm.

**13.** The process according to any of the preceding claims, wherein the fixed catalyst bed comprises three successive reaction zones, and

(i) the first reaction zone comprises shaped catalyst bodies having an external diameter of 5 mm and an internal diameter of 2 mm,
(ii) the second reaction zone comprises shaped catalyst bodies having an external diameter of 4 mm and an internal diameter of 2 mm, and
(iii) the third reaction zone comprises shaped catalyst bodies having an external diameter of 5 mm and an internal diameter of 2 mm, where the height of the shaped catalyst bodies in each case is 2.5 mm or 3 mm.

**14.** The process according to any of the preceding claims, wherein one or more reaction zones in the fixed catalyst bed comprise mixtures of shaped catalyst bodies and inert material, where steatite is used as the inert material and the inert material is in the form of hollow cylindrical shaped bodies, the dimensions of which correspond essentially to the external diameter, height and internal diameter of the shaped catalyst bodies used in the respective reaction zone.

**15.** The process according to any of the preceding claims for preparation of acrolein by gas phase oxidation of propene.

**Revendications**

**1.** Procédé pour la préparation d'un aldéhyde $\alpha,\beta$-insaturé et/ou d'un acide carboxylique $\alpha,\beta$-insaturé par oxydation en phase gazeuse d'un alcène avec de l'oxygène moléculaire sur un lit fixe catalytique, qui comprend des corps façonnés catalytiques cylindriques creux en vrac, dont la masse active est un oxyde multimétallique, **caractérisé en ce que**

(i) le lit fixe catalytique comprend au moins trois zones de réaction consécutives ;
(ii) la température locale la plus élevée du lit fixe catalytique ne survient pas dans la zone de réaction la plus proche de la sortie du réacteur ;
(iii) la température locale la plus élevée du lit fixe catalytique ne survient pas dans la zone de réaction la plus proche de l'entrée du réacteur ; et
(iv) la valeur WS selon l'équation ci-dessous dans la zone de réaction dans laquelle la température locale la plus élevée du lit fixe catalytique survient est inférieure à celle dans les autres zones de réaction,

$$WS = \frac{AD\text{-}ID}{2}$$

dans laquelle AD représente le diamètre externe et ID représente le diamètre interne du corps façonné catalytique.

**2.** Procédé selon la revendication 1, **caractérisé en ce que** l'oxyde multimétallique comprend au moins les éléments fer, bismuth et au moins un des éléments molybdène et tungstène.

**3.** Procédé selon la revendication 2, **caractérisé en ce que** l'oxyde multimétallique correspond à la formule (I),

$$Mo_{12}Bi_aFe_bX^1_cX^2_dX^3_eX^4_fO_n \qquad (I),$$

dans laquelle

$X^1$ représente nickel et/ou cobalt,
$X^2$ représente thallium, un métal alcalin et/ou un métal alcalino-terreux,
$X^3$ représente zinc, phosphore, arsenic, bore, antimoine, étain, cérium, plomb, vanadium, chrome et/ou tungstène,
$X^4$ représente silicium, aluminium, titane et/ou zirconium,
a représente un nombre dans la plage de 0,2 à 5,
b représente un nombre dans la plage de 0,01 à 10,
c représente un nombre dans la plage de 0 à 10,
d représente un nombre dans la plage de 0 à 2,

## EP 3 068 754 B1

e représente un nombre dans la plage de 0 à 8,

f représente un nombre dans la plage de 0 à 10, et

n représente un nombre qui est déterminé par la valence et la fréquence des éléments différents de l'oxygène dans (I) ;

ou correspond à la formule (II)

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad \text{(II)},$$

dans laquelle

$Y^1$ représente bismuth ; ou bismuth et au moins un des éléments tellure, antimoine, étain et cuivre,

$Y^2$ représente molybdène ; ou tungstène ; ou molybdène et tungstène,

$Y^3$ représente un métal alcalin, thallium et/ou samarium,

$Y^4$ représente un métal alcalino-terreux, nickel, cobalt, manganèse, zinc, étain, cadmium et/ou mercure,

$Y^5$ représente fer ; ou fer et au moins un des éléments vanadium, chrome et cérium,

$Y^6$ représente phosphore, arsenic, bore, antimoine et/ou bismuth,

$Y^7$ représente un métal des terres rares, titane, zirconium, niobium, tantale, rhénium, ruthénium, rhodium, cuivre, argent, or, aluminium, gallium, indium, silicium, germanium, plomb, thorium et/ou uranium,

$Y^8$ représente molybdène ; ou tungstène ; ou molybdène et tungstène,

a' représente un nombre dans la plage de 0,01 à 8,

b' représente un nombre dans la plage de 0,1 à 30,

c' représente un nombre dans la plage de 0 à 4,

d' représente un nombre dans la plage de 0 à 20,

e' représente un nombre supérieur à 0 dans la plage de 0 à 20,

f' représente un nombre dans la plage de 0 à 6,

g' représente un nombre dans la plage de 0 à 15,

h' représente un nombre dans la plage de 8 à 16,

x' et y' représentent des nombres déterminés par la valence et la fréquence des éléments différents de l'oxygène dans (II) et

p et q représentent des nombres dont le rapport p/q vaut 0,1 à 10.

**4.** Procédé selon l'une quelconque des revendications précédentes, **caractérisé en ce que** le volume géométrique du corps façonné catalytique est inférieur 80 mm$^3$.

**5.** Procédé selon l'une quelconque des revendications précédentes, **caractérisé en ce que** l'activité catalytique volumique spécifique de la zone de réaction dans laquelle la température locale la plus élevée du lit fixe catalytique survient est inférieure à celle dans les autres zones de réaction.

**6.** Procédé selon l'une quelconque des revendications précédentes, **caractérisé en ce que** la densité massique active catalytique de la zone de réaction dans laquelle la température locale la plus élevée du lit fixe catalytique survient est inférieure à celle dans les autres zones de réaction.

**7.** Procédé selon l'une quelconque des revendications précédentes, **caractérisé en ce que** le diamètre externe des corps façonnés catalytiques dans la zone de réaction dans laquelle la température locale la plus élevée du lit fixe catalytique survient est inférieur au diamètre externe des corps façonnés catalytiques dans les autres zones de réaction et le diamètre interne des corps façonnés catalytiques est identique dans toutes les zones de réaction ; ou **en ce que** le diamètre interne des corps façonnés catalytiques dans la zone de réaction dans laquelle la température locale la plus élevée du lit fixe catalytique survient est supérieur au diamètre interne des corps façonnés catalytiques dans les autres zones de réaction et le diamètre externe des corps façonnés catalytiques est identique dans toutes les zones de réaction.

**8.** Procédé selon l'une quelconque des revendications précédentes, **caractérisé en ce que** le lit fixe catalytique comprend trois zones de réaction consécutives,

(i) la première zone de réaction représentant 2 à 5% du volume du lit fixe catalytique,

(ii) la deuxième zone de réaction représentant 25 à 45% du volume du lit fixe catalytique et

(iii) la troisième zone de réaction représentant 50 à 73% du volume du lit fixe catalytique.

25

**9.** Procédé selon l'une quelconque des revendications précédentes, **caractérisé en ce que** le lit fixe catalytique comprend trois zones de réaction consécutives,

(i) la première zone de réaction présentant 70 à 90% de l'activité catalytique volumique spécifique de la troisième zone de réaction et
(ii) la deuxième zone de réaction présentant 50 à 70% de l'activité catalytique volumique spécifique de la troisième zone de réaction et

**10.** Procédé selon l'une quelconque des revendications précédentes, **caractérisé en ce que** le lit fixe catalytique comprend trois zones de réaction consécutives,

(i) la première zone de réaction présentant 70 à 90% de la densité massique active catalytique de la troisième zone de réaction et
(ii) la deuxième zone de réaction présentant 50 à 70% de la densité massique active catalytique de la troisième zone de réaction.

**11.** Procédé selon l'une quelconque des revendications précédentes, **caractérisé en ce que** les corps façonnés catalytiques cylindriques creux présentent un diamètre externe de 3 à 5 mm, une hauteur de 2 à 4 mm et un diamètre interne de 1 à 4 mm.

**12.** Procédé selon l'une quelconque des revendications précédentes, **caractérisé en ce que** le lit fixe catalytique comprend trois zones de réaction consécutives,

(i) la première zone de réaction comprenant des corps façonnés catalytiques présentant un diamètre externe de 5 mm et un diamètre interne de 2 mm,
(ii) la deuxième zone de réaction comprenant des corps façonnés catalytiques présentant un diamètre externe de 5 mm et un diamètre interne de 3 mm et
(iii) la troisième zone de réaction comprenant des corps façonnés catalytiques cylindriques présentant un diamètre externe de 5 mm et un diamètre interne de 2 mm,

la hauteur des corps façonnés catalytiques étant à chaque fois de 2,5 mm ou 3 mm.

**13.** Procédé selon l'une quelconque des revendications précédentes, **caractérisé en ce que** le lit fixe catalytique comprend trois zones de réaction consécutives,

(i) la première zone de réaction comprenant des corps façonnés catalytiques présentant un diamètre externe de 5 mm et un diamètre interne de 2 mm,
(ii) la deuxième zone de réaction comprenant des corps façonnés catalytiques présentant un diamètre externe de 4 mm et un diamètre interne de 2 mm et
(iii) la troisième zone de réaction comprenant des corps façonnés catalytiques cylindriques présentant un diamètre externe de 5 mm et un diamètre interne de 2 mm,

la hauteur des corps façonnés catalytiques étant à chaque fois de 2,5 mm ou 3 mm.

**14.** Procédé selon l'une quelconque des revendications précédentes, **caractérisé en ce qu'**une ou plusieurs zones de réaction du lit fixe catalytique contien(nen)t des mélanges de corps façonnés catalytiques et de matériau inerte, la stéatite étant utilisée comme matériau inerte et le matériau inerte se trouvant sous forme de corps façonnés cylindriques creux dont les dimensions correspondent essentiellement au diamètre externe, à la hauteur et au diamètre interne des corps façonnés catalytiques utilisés dans la zone de réaction respective.

**15.** Procédé selon l'une quelconque des revendications précédentes pour la préparation d'acroléine par oxydation en phase gazeuse de propène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10336386 A
- EP 1350566 B1
- EP 1526123 B1
- DE 10232748 A
- EP 184790 A
- US 4656157 A
- DE 102007003076 A
- DE 4407020 A
- EP 575897 A
- DE 3338380 C
- DE 102006044520 A
- DE 10049873 A
- WO 2008087116 A
- DE 102008042060 A
- DE 102007004961 A
- WO 2005030393 A
- US 20050131253 A
- WO 2007017431 A
- DE 102007005606 A
- DE 102008040093 A

- DE 10046957 A
- WO 0224620 A
- DE 4431957 A
- EP 700714 A
- EP 700893 A
- EP 468290 B
- DE 19910506 A
- DE 10313213 A
- DE 10313208 A
- EP 1106598 A
- WO 2007082827 A
- EP 873783 A
- EP 1270065 A
- US 7534339 B2
- DE 10337788 A
- DE 102009047291 A
- DE 10200804093 A
- DE 102011084040 A1
- EP 1547994 A

**In der Beschreibung aufgeführte Nicht-Patentliteratur**

- Die Tablette. **W. A. RITSCHEL ; A. BAUER-BRANDL.** Handbuch der Entwicklung, Herstellung und Qualitätssicherung. Verlag Aulendorf, 2002

- Die Bibliothek der Technik. vol. 229
- Thermogravimetrische Materialfeuchtebestimmung. Grundlagen und praktische Anwendungen. Verlag moderne Industrie